# EUROPEAN PATENT APPLICATION

(11) **EP 2 530 158 A1**
(43) Date of publication of application: **05.12.2012**
(21) Application number: 11736646.8
(22) Date of filing: 28.01.2011
(51) Int. Cl.: C12N 15/29, C12N 15/57, C07K 14/415, C12N 15/63, C12N 15/64, C12N 15/79, C12N 15/82

(54) **GENES ENCODING PROTEINS ABLE TO ENHANCE HEAT RESISTANCE OF PLANTS AND MICROORGANISM AND USES THEREOF**

(30) Priority: 28.01.2010 CN 201010300890
(71) Applicant: Sichuan Biodesign Gene Engineering Co., Ltd., Chengdu, Sichuan 610000 (CN)
(72) Inventor: YANG, Yi, Sichuan 610000 (CN)
(74) Representative: McKeown, Yvonne Mary
(86) International application number: PCT/CN2011/070745
(87) International publication number: WO 2011/091764

(57) **Abstract**

Genes encoding proteins able to enhance heat resistance of plants and microorganisms, proteins encoded by the same and uses thereof are provided, wherein the genes contain nucleotide sequences encoding the following peptide fragments: N'-CRICQE X₇₋₄₅ PCAC X₆ AHR X₁ CVQ X₁₃₋₂₇-C', wherein X represents any amino acid and the subscript represents the number of amino acid.

## Description

### Technical field

The present invention belongs to molecular biology field. And more specifically, the invention relates to genes encoding proteins able to enhance heat resistance of plants and microorganisms, proteins encoded by the same and uses thereof.

### Background art

Because of the increased CO₂ emission, greenhouse effect on the earth is growing worse and leads to global warming. It is estimated that the global average temperature will increase by 1.4-5.8°C in the next 100 years. Global warming gradually deteriorates the agricultural ecological environment. It is predicted that climate warming may lead to 17% of crop yield reduction. One research from IRRI (International Rice Research Institute) proved that during 1998-2003, the crop yield was decreased by 10% with the temperature elevated by 1°C. In China, experts believe that by 2050, the nationwide average temperature will increase by 2.2°C. Plants growing under natural conditions are all affected by the elevated temperature and grow more slowly. Some major crops, such as rice and corn, are especially easy to be influenced by hot weather during earing and pustulation, and result in crop yield reduction. On the other hand, according to FAO (Food and Agriculture Organization of the United Nations), the world population will exceed 10 billions by 2050. With further increased world population, there will be more and more pressure on agriculture and worldwide food shortage will be a long-lasting problem. Being affected by global warming, lots of herbaceous plants will grow more slowly and even die, thus breaking ecosystem balance. Therefore, scientists all over the world are taking great efforts in searching for heat-tolerance relevant plants genes. So far from now, only a few heatshock protein genes and transcription factors thereof are found to be relevant with heat tolerance, while none of a single gene was reported to be capable of increasing heat tolerance of bacteria and plants. It is now of great demands in the art to find genes able to enhance heat resistance of plants.

Ubiquitin, which is named because of its wide distribution in various kinds of cells, is a highly conserved polypeptide consisting of 76 amino acid residues. Ubiquitin-labeled proteins will be specifically recognized and rapidly degraded. As is shown in studies, ubiquitin-mediated protein degradation is an important physiological and biochemical process for organisms to maintain normal growth as well as growth under stresses.

Ubiquitination of protein mainly occurs to the side chain of lysine residue and is usually a multimerization process. Multi-ubiquitinated proteins will be recognized and degraded by proteasome. There are three crucial enzymes that mediate such a multi-ubiquitination process, including ubiquitin activating enzyme E1, ubiquitin conjugating enzyme E2 and ubiquitin ligase E3. The mechanism of those enzymes is now substantially illustrated as follows: firstly, a thioester bond is formed between the Cys residue of the active center of E1 and the C-terminal of ubiquitin, to activate the individual free ubiquitin (wherein ATP is needed); then, the activated ubiquitin is delivered from E1 to E2; finally, specific substrate and E2 are collected by E3, which mediates the transferring of ubiquitin from E2 to the target protein. Since the process of recognition and degradation of ubiquitinated protein by proteasome is non-specific, the role of E3 is extremely important in the protein degradation process to determine the specificity of the reaction. Usually, ubiquitination involves one E1, several E2 and many E3. The E3 family discoved so far has two types, including the majority of RING-finger family and the HECT family.

### Disclosure of the invention

The technical problem to be solved in the invention is to provide genes (also called heat-resisting genes in the invention) encoding proteins (also called heat-resisting proteins in the invention) that are able to enhance heat resistance of plants and microorganisms.

Said genes encoding proteins that are able to enhance heat resistance of plants or microorganisms comprise nucleotide sequences encoding peptides with structures as shown in
N'- CRICQE X₇₋₄₅ PCAC X₆ AHR X₁ CVQ X₁₃₋₂₇ -C' (SEQ ID NO: 68), wherein X represents any amino acid and the subscript represents the number of amino acid.

Furthermore, said peptides have amino acid sequences of:
N'- CRICQEED X₃₋₂₀ NL X₃₋₂₀ PCAC X₂ SLK X₁ AHR X₁ CVQRWC X₁₀₋₂₄ -C' (SEQ ID NO: 69), wherein X represents any amino acid residue and the subscript represents the number of amino acid.

Wherein, the forementioned proteins are ubiquitin ligases (E3).

Wherein, the forementioned proteins are transmembrane proteins, and comprise transmembrane domains.

Wherein, the forementioned genes are derived from plant genomes.

Wherein, the forementioned plants are selected from arabidopsis (*Arabidopsis thaliana* L.), rice (*Oryza sativa* L.), corn (*Zea mays* L.) or castor oil plant (*Ricinus communis* L.).

Wherein, the forementioned genes (1): comprise nucleotide sequences shown in SEQ ID NO: 39, SEQ ID NO: 1, SEQ ID NO: 45, SEQ ID NO: 47 or SEQ ID NO: 57;

Or (2): the forementioned genes comprise nucleotide sequences derived from substitution, deletion or addition of at least one nucleotide in any one of said nucleotide sequences in (1), while encoding proteins with the same or similiar function.

Furthermore, said genes comprise (1): nucleotide sequences of sites 158-1018 in SEQ ID NO: 57, sites 96-848 in SEQ ID NO: 45, sites 291-1127 in SEQ ID NO: 47, or sites 68-946 in SEQ ID NO: 39;

Or (2): said genes comprise nucleotide sequences derived from substitution, deletion or addition of at least one nucleotide in any one of said nucleotide sequences in (1), while encoding proteins with the same or similiar function.

The invention also provides proteins encoded by the forementioned genes.

The invention further provides proteins that enhance the heat resistance of plants or microorganisms. Such proteins comprise peptides with structures as shown in:
N'- CRICQE X₇₋₄₅ PCAC X₆ AHR X₁ CVQ X₁₃₋₂₇ -C', wherein X represents any amino acid and the subscript represents the number of amino acid.

Furthermore, said peptides have amino acid sequences of:
N'- CRICQEED X₃₋₂₀ NL X₃₋₂₀ PCAC X₂ SLK X₁ AHR X₁ CVQRWC X₁₀₋₂₄ -C', wherein X represents any amino acid.

Wherein, the forementioned proteins are transmembrane proteins comprising transmembrane domains. The forementioned proteins comprise 1-6 transmembrane domains. Furthermore, said protein comprises 2-3 transmembrane domains.

Wherein, the structure of the forementioned transmembrane domain is at least one of N'- A X₂₋₆ CRS X₂₋₈ LIL X₂₋₄ LL X₁₋₄ LR X₁₋₁₀ -C' (SEQ ID NO: 70), or N'- L X₂₋₄ R X₁₋₅ GFLL X₁₋₇ YIMAW X₁₋₁₅ -C' (SEQ ID NO: 71), wherein X represents any amino acid and the subscript represents the number of amino acid. Wherein, there is a flexible connection between each of the forementioned transmembrane domains. Wherein, the forementioned transmembrane domain is close to the C-terminal. Wherein, the forementioned transmembrane domain is flexibly connected to a zinc finger domain. Wherein, the forementioned protein also has a signal peptide at the N-terminal. Wherein, the forementioned signal peptide is a membrane localization signal peptide.

Wherein, the forementioned proteins are ubiquitin ligases.

Wherein, the forementioned proteins are derived from plants.

Wherein, the forementioned proteins comprise amino acid sequences encoded by nucleotide sequences shown in SEQ ID NO: 1 (the amino acid sequence encoded by which is shown in SEQ ID NO: 2), sites 158-1018 in SEQ ID NO: 57 (the amino acid sequence encoded by which is shown in SEQ ID NO: 58), sites 96-848 in SEQ ID NO: 45 (the amino acid sequence encoded by which is shown in SEQ ID NO: 46), sites 291-1127 in SEQ ID NO: 47 (the amino acid sequence encoded by which is shown in SEQ ID NO: 48) or sites 68-946 in SEQ ID NO: 39 (the amino acid sequence encoded by which is shown in SEQ ID NO: 40);

Or (2): said protein comprises amino acid sequences derived from substitution, deletion or addition of at least one amino acid in any one of amino acid sequences in (1), while possessing with the same or similiar function.

The invention also provides genes encoding the forementioned heat-resisting proteins.

The invention also provides peptides comprising domains as shown in N'- CRICQE X₇₋₄₅ PCAC X₆ AHR X₁ CVQ X₁₃₋₂₇ -C', wherein X represents any amino acid and the subscript represents the number of amino acid.

Furthermore, said domains comprise amino acid sequences of N'- CRICQEED X₃₋₂₀ NL X₃₋₂₀ PCAC X₂ SLK X₁ AHR X₁ CVQRWC X₁₀₋₂₄ -C', wherein X represents any amino acid residue and the subscript represents the number of amino acid.

Wherein, the forementioned peptides are derived from plants. Said plants may be selected from arabidopsis, rice, corn or castor oil plant.

Wherein, the forementioned peptides may form zinc fingers, i.e., domains as shown in N'-CRICQE X₇₋₄₅ PCAC X6 AHR X1 CVQ X₁₃₋₂₇ -C' or N'- CRICQEED X₃₋₂₀ NL X₃₋₂₀ PCAC X₂ SLK X₁ AHR X₁ CVQRWC X₁₀₋₂₄ -C' may form zinc fingers, wherein X represents any amino acid and the subscript represents the number of amino acid.

Wherein, the forementioned peptides may enable proteins to enhance the heat resistance of plants or microorganisms.

Furthermore, the forementioned stress resistance is heat resistance. Moreover, said protein is an ubiquitin ligase.

Moreover, the forementioned peptides comprise sequences as follows:
(1): amino acid sequences shown in SEQ ID NO: 63, SEQ ID NO: 64, SEQ ID NO: 65, SEQ ID NO: 66 or SEQ ID NO: 67;

Or, amino acid sequences derived from substitution, deletion or addition of at least one amino acid in any one of amino acid sequences in (1), while possessing with the same or similiar function.

Wherein, the forementioned "at least one" means one or several (less than 10). The forementioned "same or similiar function" means the ability to enhance the heat resistance of plants or microorganisms.

The invention also provides nucleotide sequences encoding the forementioned peptides.

The invention also provides a method of modifying genes encoding non- heat-resisting proteins into those encoding heat-resisting ones, comprising the step of inserting forementioned peptide-encoding nucleotide sequences into genes encoding non- heat-resisting proteins, or substituting one section of nucleotide sequences therein. Certainly, such an insertion or substitution occurs between, not inside, codons, and therefore the modified gene still has the ability to encode an entire protein. That is to say, the method comprise the step of inserting forementioned peptide-encoding nucleotide sequences into genes encoding non- heat-resisting proteins, or substituting one section of nucleotide sequences therein; and such an insertion or substitution occurs between codons, therefore the modified gene still has the ability to encode an entire protein.

Wherein, the forementioned substituted nucleotide sequence is the one that encoding zinc finger domain. That is to say, the nucleotide sequence substituted from the forementioned gene encoding non- heat-resisting protein is the one that encoding zinc finger domain.

Wherein, the forementioned non- heat-resisting proteins are ubiquitin ligases (E3 ligase).

Based on the forementioned parts, the invention further provides a method for enhancing the heat resistance of plants, or a method for preparing plants with high heat resistance. Such a method includes steps as follows:
a). operably linking the forementioned gene encoding heat-resisting protein into the downstream of the expression and regulation sequence of a vector, to form a recombinant vector for the expression of said gene;
b). transforming the recombinant vector of step a) into plant cells; and
c). selecting and obtaining transformed cells, followed by regenerating the transformed cells to form transgenic plants or offsprings thereof, said offsprings include plant seeds or plant tissues. Furthermore, the stress resistance in the forementioned method is heat resistance.

The invention also provides vectors comprising the forementioned gene sequences or nucleotide sequences, wherein, said vectors are expression vectors.

Furthermore, said expression vectors are eukaryotic expression vectors.

The invention also provides host cells comprising the forementioned vectors. Said host cells are mainly plant cells or microorganisms.

Said recombinant vectors of the invention are obtained by inserting genes into vectors. The forementioned vectors may be selected from various vectors known in the art, especially eukaryotic expression vectors (e.g. pBI121 or pCAMBIA2301). Host plant cells can be transformed with the forementioned recombinant vectors of the invention, and the transformed cells can be screened under various forementioned stresses.

In the present invention, statements like "nucleotide sequences derived from substitution, deletion or addition of at least one nucleotide in SEQ ID NO: 1" generally refer to nucleotide sequences encoding polypeptides that possess with the activity of the protein encoded by SEQ ID NO: 1, and the degenerate sequences thereof. Said degenerate sequences are sequences with one or more codons being substituted by degenerate codons encoding the same amino acid. Because of the codon degeneracy, a degenerate sequence exhibiting as low as 89% of homology to SEQ ID NO: 1 may encode the sequence encoded by SEQ ID NO: 1 as well. The term of "nucleotide sequences derived from substitution, deletion or addition of at least one nucleotide in SEQ ID NO: 1" also includes nucleotide sequences that can hybridize with SEQ ID NO: 1 under moderate stringent conditions, preferably under high stringent conditions. The term also includes nucleotide sequences exhibiting at least 80%, more preferably at least 90%, and most preferably at least 95% of homology to the nucleotide sequence of SEQ ID NO: 1. The same function in the present invention means enhancing heat resistance of plants or microorganisms.

The term also includes variants of the open reading frame sequence of SEQ ID NO: 1 that can encode proteins having the same function as natural SEQ ID NO: 1. Such variants include (but not limit to): deletion, insertion and/or substitution of several nucleotides (normally 1-90, preferably 1-60, more preferably 1-20 and most preferably 1-10), as well as addition at 5' and/or 3' terminals of several nucleotides (normally less than 60, preferably less than 30, more preferably less than 10 and most preferably less than 5).

In the present invention, statements like "amino acid sequences derived from substitution, deletion or addition of at least one amino acid in said amino acid sequences" include, but not limit to, deletion, insertion and/or substitution of several amino acids (normally 1-50, preferably 1-30, more preferably 1-20 and most preferably 1-10), as well as addition at C- and/or N-terminals of several amino acids (normally less than 20, preferably less than 10 and more preferably less than 5). For example, in said proteins, substitutions by amino acids with similiar properties usually do not change the function of the protein. Another example is that the addition at C- and/or N- terminals of one or more amino acids usually does not change the protein function, either. The term also includes active fragments and derivatives of said proteins. The same function in the present invention means enhancing heat resistance of plants or making other proteins capable of enhancing heat resistance of plants or microorganisms.

Statements like "amino acid sequences derived from substitution, deletion or addition of at least one amino acid in said amino acid sequences" also include, but not limited to polypeptides having at most 10, preferably at most 8 and more preferably at most 5 of amino acids substituted by those with similar properties, namely, polypeptides with conserved variation. Such polypeptides with conserved variation are obtained most preferably from substitutions according to Table 1.

**Table 1 Substitutions of amino acids**

| Original residues | Representative substitutions | Preferable substitutions |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also includes analogues of the claimed proteins or polypeptides. Such analogues may be different from the natural polypeptide of SEQ ID NO: 2 in amino acid sequence, or in modifications that will not change the sequence, or both. Such polypeptides include natural or induced genetic variants. The induced variants could be obtained with various techniques, such as random mutagenesis by radiation or exposing to mutagens, as well as site-directed mutagenesis or other known molecular biology techniques. Said analogues also include those comprising residues different from natural L- amino acids (e.g. D- amino acids), and those comprising non-naturally existed or synthetic amino acids (e.g. β, γ-amino acids). It should be appreciated that proteins or polypeptides of the present invention are not limited to the above-mentioned exemplary representatives.

The modification (normally without changing the primary structure) includes chemical derivatization of polypeptides *in vivo* or *in vitro,* such as acetylation or carboxylation. The modification also includes glycosylation, such as polypeptides produced from glycosylation in polypeptide synthesis, or from processing or reprocessing steps, which could be achieved by exposing polypeptide to enzymes performing glycosylation (e.g. glycosylase or deglycosylase of mammals). The modification also includes sequences comprising phospho-amino acid residues (e.g. phosphotyrosine, phosphoserine or phosphothreonine). Also included are polypeptides modified to have the property of enhanced proteolysis resistance or improved solubility.

Said "operably-linked" in the present invention refers to conditions that certain parts of linear DNA sequence can affect activities of the others on the same linear DNA sequence. For example, if a signal peptide DNA is expressed as a precursor and participates the secretion of a polypeptide, then the signal peptide DNA (that secretes leading sequence) is operably-linked to the polypeptide DNA; if a promoter controls the transcription of a sequence, then it is operably-linked to the coding sequence; or if a ribosome binding site is placed at a position that it could be translated, then it is operably-linked to a coding sequence. Generally speaking, "operably-linked" means being contigeous, and for the secretion of leading sequence it means being contigeous in reading frames.

The invention shows advantages as follows: it provides genes encoding heat-resisting proteins, and proteins encoded thereof; it also provides polypeptides functioning similiarly, and genes thereof; and it further provides uses of these genes and proteins for the enhancement of heat resistance of plants and microorganisms. The invention provides new alternative genes for the enhancement of heat resistance of plants and microorganisms and therefore promises a good application prospect.

### Description of drawings

Fig. 1 Growth curve of E. coli transformed with AtTR1 gene and mutants thereof. In the figure, AtTR1 represents E. coli transformed with over expressed AtTR1 gene, MT1-6 respectively represents E. coli transformed with over expressed mutant AtTR1 genes, and WT is non-transgenic E. coli.
Fig. 2 Growth curve of E. coli transformed with RcTR1 gene, the homologous gene of AtTR1 in castor oil plant.
Fig. 3 Growth curve of E. coli transformed with OsTR1-1 and OsTR1-2 genes, the homologous genes of AtTR1 in rice.
Fig. 4 Growth curve of E. coli transformed with ZmTR1 gene, the homologous gene of AtTR1 in corn.
Fig. 5 Growth curve of E. coli with the transmembrane domain of AtTR1 protein substituted or deleted.
Fig. 6 Growth curve of E. coli with the transmembrane domain of OsTR1-1 protein substituted or deleted.
Fig. 7 Growth curve of E. coli with the transmembrane domain of ZmTR1 protein substituted or deleted.
Fig. 8 Detection of E3 ubiquitin ligase activity of AtTR1 protein (+ for added; - for not added)
Fig. 9 Detection of E3 ubiquitin ligase activity of OsTR1-1 protein (+ for added; - for not added)
Fig. 10 Detection of E3 ubiquitin ligase activity of ZmTR1 protein (+ for added; - for not added)

### Embodiments

The following experiments were performed according to, if not specially defined, routine conditions known by those skilled in the art, such as in Sambrook and Russell, Molecular Cloning: A Laboratory Manual (3rd ed). (New York: Cold Spring Harbor Laboratory Press, 1989), or conditions suggested by manufacturers. In the following examples, vectors pET28, pGEX-2T, pGEM-T and strain BL21 were purchased from Qiagen corp., and strain EHA105 and vector pBI121 were from Clontech corp.

In recent years, it is a hotspot to improve plants with genetic engineering techniques, and there is one choice to enhance stress resistances of plants and cultivate plant lines of resistance by genetic engineering techniques. However, there is seldom report of a single gene that could comprehensively enhance several of resistances for plants and microbes to abiotic stresses. The inventor has cloned an AtTR1 gene from arabidopsis that could enhance heat resistance of plants.

Study showed that, protein expressed by said AtTR1 gene is possessed with ubiquitin E3 ligase activity of the ubiquitin protein system; and by transforming arabidopsis, brassica, and E. coli with the AtTR1 gene, heat resistances in all transgenic organisms were enhanced. Therefore, the gene might be used in the enhancement of heat resistance of plants and microorganisms to reduce the crop failure from high temperature, as well as lowering costs, thus promising economic significance and application prospect.

Mutations were performed to some conserved amino acids of AtTR1 protein in order to study the mechanism of the AtTR1 gene and search for critical functional sites of AtTR1 protein.

**Table 2 Alignment of AtTR1 and the MARCH family proteins**

| Proteins | Critical sequences (the Ring structures) |
|---|---|
| AtTR1 | 69 **C**RI**C**QE.EDSTKNLEAP**C**A**C**NGSLKYA**H**RK**C**VQRWCNEKGDIT**C**EI**C** 114 |
| MARCH2 | 64 **C**RI**C**HE.GANGECLLSP**C**G**C**TGTLGAV**H**KS**C**LEKWLSSSNTSY**C**EL**C** 109 |
| MARCH4 | 163 **C**RI**C**FQ.GPEQGELLSP**C**R**C**DGSVKCT**H**QP**C**LIKWISERGCWS**C**EL**C** 208 |
| MARCH6 | 9 **C**RV**C**RSEGTPEKPLYHP**C**V**C**TGSIKFI**H**QE**C**LVQWLKHSRKEY**C**EL**C** 55 |

The alignment (see Table 2) of protein structures showed that AtTR1 protein shared similar structure with MARCH (Membrane Associated Ring-CH), a protein involved in the human immune regulation, wherein both proteins comprised a typical Ring-CH domain. Mutations were performed at several critical sites shown in the alignment. Results showed that these mutants exhibited changed heat resistance, higher or lower than that of the non-transgenic, but all lower than that oftrans-AtTRl gene, showing that these critical sites determined the activity of AtTR1 protein of enhancing stress resistance of organisms.

Based on the forementioned study results, several homologous genes of the AtTR1 gene were found in other plants. Said genes comprised some highly conserved regions and they too were possessed with the function of enhancing heat resistance of plants (see Table 3 for core sequence analysis).

**Table 3 Alignment of functional sites of AtTR1 and homologous proteins thereof**

| | | |
|---|---|---|
| Arabidopsis | 60 EEPLLQSVECRICOEEDSTKNLEAPCACNGSLKYAHRKCVORWCNEKGDITCEICHQPYQ 119 | |
| Castor oil plant | 60 EEPLLQTMFCRICOEEDSINNLEAPCACSGSLKFAHRKCVORWCNEKGDITCEICHQPYQ 119 | |
| Corn | 59 E PLIQAAECRICOEEDSVKNLEKPCACSGSLKYAHRACVORWCNEKGDTTCEICHEEYK 118 | |
| Rice 1 | 23 TGALIGMVECRICOEEDLAKNTLESPCACSGSLKYAHRECVORWCNEKGDITCEICHVSYK 82 | |
| Rice 2 | 50 EEPLIQAAECRICOEEDSIKNLEKPCACSGSLKYAHRACVORWCNEKGDITCETCHEQYK 109 | |
| | RINGv region | |
| Arabidopsis | 180 AAFCRSAALILMALLLRDALNLTTNPDDEDDPTAFFSLFLLRAAGFLLPCYIMAWAIGI 239 | |
| Castor oil plant | 181 AAFCRSAALILMALLLLRHAMSLTG DSDEDASTFFSLLRRAAGFLLPCYIMAWAISI 238 | |
| Corn | 175 AAFCRSAALILMALLLLRHALSMSDNEGNDDDASTIFSLFLLRAAGFPCYIMAWIFSI 234 | |
| Rice 1 | 145 AAFCRSIFLILMALLLLRHTLTITSSDDE DDASAIFSLFLLRAAGFLLPCYIMAWAISI 203 | |
| Rice 2 | 163 AAFCRSAALILMALLLLRHALSISDNEGDDDASTMFSLFLLRAAGFLLPCYIMAWIFSI 221 | |
| | TMD transmembrane domain | TMD transmembrane domain |

It was confirmed that a conserved RINGv region comprising amino acid sequence as follows was essential for heat resistance:
N-terminal - *C* RI*C* QE X₇₋₄₅ P*C* A*C* X₆ AH R X₁ *C* VQ X₁₃₋₂₇ - C-terminal

Furthermore, said region comprised:
N'- *C* RI*C* QEED X₃₋₂₀ NL X₃₋₂₀ P*C* A*C* X₂ SLK X₁ AH R X₁ *C* VQRWC X₁₀₋₂₄ -C'

The italic amino acids referred to sites that could not be changed according to the mutation experiment, and if mutated, the heat resistance would be lost or reduced; wherein X represents any amino acid (preferably natural amino acids) and the subscript represents the number of amino acid.

**Table 4 Patterns of common zinc finger proteins**

| 1 | | 2 | | 3 | | 4 | | 5 | | 6 | | 7 | | 8 | |
|---|---|---|---|---|---|---|---|---|---|---|---|---|---|---|---|
| C | X₂ | C | X₉₋₃₉ | C | X₁₋₂ | H | X₂₋₃ | C | X₂ | C | X₄₋₄₈ | C | X₂ | C | RING |
| C | X₂ | C | X₉₋₃₉ | C | X₁₋₂ | H | X₂₋₃ | H | X₂ | C | X₄₋₄₈ | C | X₂ | C | RING-H2 |
| C | X₂ | C | X₁₀₋₄₅ | C | X₁ | C | X₇ | H | X₂ | C | X₁₁₋₂₅ | C | X₂ | C | RINGv(RING-CH) |
| C | X₁₋₂ | C | X₇₋₂₁ | C | X₂₋₄ | C | X₄₋₅ | H | X₂ | C | X₁₀₋₄₆ | C | X₂ | C | PHD-LAP |

It was shown in Table 4 that, the amino acids at forementioned 1, 2 and 5, 6 sites formed a zinc finger, wherein x could be an arbitrary amino acid. Line 3 showed a specific zinc finger (RINGv) of proteins of the invention, which was specific in plants, and closely related to heat resistance of proteins of the invention.

### Example 1 Cloning of heat-risisting AtTR1 gene from arabidopsis and construction of over expression recombinant vector

1. The AtTR1 gene was selected from arabidopsis with a nucleotide sequence as shown in SEQ ID NO: 1 from the sequence list. Primers were designed according to the nucleotide sequence as shown in SEQ ID NO: 1, as follows:
Upstream primer (SEQ ID NO: 3): 5'-ATGGCTGATCATTTGAGTTTATGT-3',
Downstream primer (SEQ ID NO: 4): 5'-TCAAACTGGTGTTGGGACATTGGATA-3'.

The nucleotide sequence was then amplified by PCR from arabidopsis cDNA. The PCR product was purified (see manuals of PCR product purification of Qiagen corp.) and sequenced to obtain gene fragments of the sequence of SEQ ID NO: 1.

2. Primers were designed according to the nucleotide sequence of SEQ ID NO: 1, as follows:
Upstream primer (SEQ ID NO: 5): 5'-CGCGGATCC ATGGCTGATCATTTGAGTTTATGT-3',
Downstream primer (SEQ ID NO: 6): 5'-CCGGAGCTC TCAAACTGGTGTTGGGACATTGGATA-3'.

Restriction enzyme cutting sites were added by PCR to the entire SEQ ID NO: 1 nucleotide sequence amplified from arabidopsis cDNA. The PCR product was purified (see manuals from Qiagen corp.), enzymatic digested by BamH1 and Sac1, recovered from the gel, and ligated into the vector pBI121 (ligation sites: BamH1 and Sac1), to obtain the over expressed recombinant plasmid of SEQ ID NO: 1.

### Example 2 Site-directed mutagenesis of AtTR1 gene and the construction of over expression recombinant vectors comprising mutated genes

1. Several sites were randomly selected and primers comprising mutated sites were designed according to SEQ ID NO: 1 (mutations were indicated by underlines), to construct sequences of site-directed mutagenesis (Stratagene Corp., QuikChange® Site-Directed Mutagenesis Kit).

Primers for amino acid sequence comprising mutation at site 69:
Upstream primer (SEQ ID NO: 7): 5'-TTCTC CAATC TGTTG AGAGT CGTAT TTGCC-3'
Downstream primer (SEQ ID NO: 8): 5'-GGCAA ATACG ACTCT CAACA GATTG GAGAA-3'

Primers for amino acid sequence comprising mutation at site 72:
Upstream primer (SEQ ID NO: 9): 5'-AGTGT CGTAT TGGCC AAGAG GAAGA TAGTA CT-3'
Downstream primer (SEQ ID NO: 10): 5'-AG TACTA TCTTC CTCTT GGCCA ATACG ACACT-3'

Primers for amino acid sequence comprising mutation at site 85:
Upstream primer (SEQ ID NO: 11): 5'-CTTGAAGCTC CTAGT GCTTG TAA-3'
Downstream primer (SEQ ID NO: 12): 5'-TTA CAAGC ACTAG GAGCT TCAAG-3'

Primers for amino acid sequence comprising mutation at site 87:
Upstream primer (SEQ ID NO: 13): 5'-GAAGC TCCTT GTGCT CGTAA TGGTA GTTTAAA-3'
Downstream primer (SEQ ID NO: 14): 5'-TT TAAAC TACCA TTACG AGCAC AAGGA GCTTC-3'

Primers for amino acid sequence comprising mutation at site 95:
Upstream primer (SEQ ID NO: 15): 5'-TAAAG TATGC TAACC GCAAG TGTGT TC-3'
Downstream primer (SEQ ID NO: 16): 5'-GA ACACA CTTGC GGTTA GCATA CTTTA-3'

Primers for amino acid sequence comprising mutation at site 98:
Upstream primer (SEQ ID NO: 17): 5'-ACCGC AAGTA TGTTC AGCGT TGGTG TA-3'
Downstream primer (SEQ ID NO: 18): 5'-TA CACCA ACGCT GAACA TACTT GCGGT-3'

Vector pET28 comprising SEQ ID NO: 1 was used as a template, and mutant plasmids was amplified by PCR, comprising respectively SEQ ID NO: 19 (SEQ ID NO: 2 N-terminal Cys69 substituted by Ser), SEQ ID NO: 20 (SEQ ID NO: 2 N-terminal Cys72 substituted by Gly), SEQ ID NO: 21 (SEQ ID NO: 2 N-terminal Cys85 substituted by Ser), SEQ ID NO: 22 (SEQ ID NO: 2 N-terminal Cys87 substituted by Arg), SEQ ID NO: 23 (SEQ ID NO: 2 N-terminal His95 substituted by Asn), and SEQ ID NO: 24 (SEQ ID NO: 2 N-terminal 98Cys substituted by Tyr).

2. The entire nucleotide sequences shown in SEQ ID NO: 19-24 were amplified from mutant plasmids with primers as follows:
Upstream primer (SEQ ID NO: 5): 5'-CGCGGATCC ATGGCTGATCATTTGAGTTTATGT-3',
Downstream primer (SEQ ID NO: 6): 5'-CCGGAGCTC TCAAACTGGTGTTGGGACATTGGATA-3'.

PCR products were purified (see manuals from Qiagen corp.), enzymatic digested by BamH1 and Sac1, recovered from the gel, and ligated into the vector pBI121 (ligation sites: BamH1 and Sac1), to obtain over expression recombinant plasmid comprising SEQ ID NO: 19-24, which were named MT1-6, respectively.

### Example 3 Characterization of the heat-resisting capacity of E. coli with over expressed AtTR1 and mutants thereof

### 1. E. coli transformation

E. coli was transformed with recombinant plasmid and plated onto LB agar containing Amp. The E. coli pET28 strain containing recombinant plasmid was obtained after sequencing.

### 2. Medium preparation

LB liquid medium was prepared with the addition of antibiotics Kan 50ug/ml and Cam 50ug/ml, and then stored in 24 tubes, each with 10ml.

### 3. Bacterial suspention preparation

Single colonies of E. coli comprising over expressed AtTR1 and the 6 mutant genes (MT 1-6) and non-transgenic E. coli were activated overnight at 37 °C.

### 4. Dropwise addition of sample bacteria

0.05 ml of activited bacteria liquid was innoculated into tubes containing LB culture medium, 3 tubes for each bacterium, followed by shaking and cultivating (37°C, 225rpm) for 3 hours.

### 5. Culture and observation

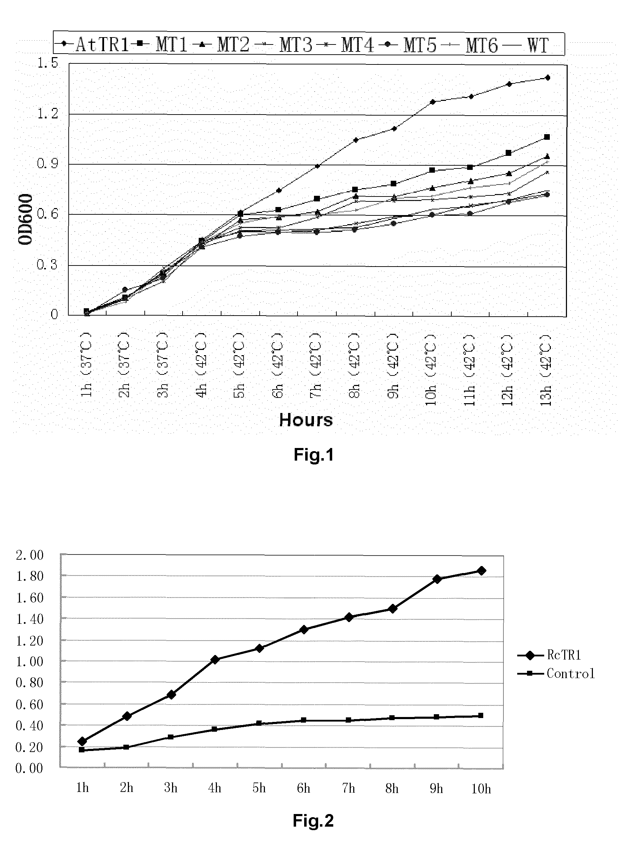

After being incubated at 37°C for 3 hours, the culture was added with isopropyl β-D-1-thiogalactopyranoside (IPTG) in a concentration of 0.1 mM, and the incubation was continued at 42°C for 10 h. The growth status of E. coli comprising over expressed AtTR1 and the 6 mutant genes (MT1-6) and non-transgenic E. coli were observed with naked eyes, and OD600 values were determined at regular times (TU-1800 ultraviolet spectrophotometer, Puxitong instrument corp., Beijing, China) to record growth curves of different E. coli, see Fig. 1 (wherein AtTR1 meant E. coli transformed with over expressed AtTR1 gene, MT1-6 respectively meant E. coli transformed with over expressed mutant AtTR1 genes, and WT meant non-transgenic E. coli).

Results showed that, although E. coli transformed with over expressed mutant AtTR1 genes exhibited some heat resistance, the slopes of their growth curves were significantly smaller than that of E. coli with over expressed AtTR1, indicating that heat resistance would be lowered after mutations at the 6 sites and such sites played an important role in the heat-resisting function of the AtTR1 protein in organisms.

### Example 4 Characterization of the heat-resisting capacity of brassica transformed with over expressed AtTR1 and mutants thereof

### 1. Transformation of Brassica napus by hypocotyl infection

### 1-1. obtaining aseptic seedlings

Plump *Brassica napus* seeds were selected and vernalized overnight at 4 °C (synchronized germination), then immersed with 70% of ethanol for 30s and 0.1% of mercuric chloride (HgCl₂) solution for 8-10 min, rinsed with sterile water for 5 times and dried with filter paper, and then plated onto MS agar medium. After cultivated at 24°C for 2-3 days in a dark chamber, the culture was exposed to illumination for 16 h/d to continue germination. The hypocotyl of aseptic seedlings (in about 7-8 days) was taken with 5-7 cm as transformation receptors.

### 1-2. Pre-culture of the hypocotyl

The *Brassica napus* hypocotyl was sliced into sections of about 7 mm and well distributed onto pre-culture medium (MS+2 mg/L of 6-BA, 1 mg/L of 2,4-D, 2.5 mg/L of AgNO₃ and 19.62 mg/L of acetosyringone) to be pre-cultivated for 2-3 days (wherein the hypocotyl became coarse).

### 1-3. Infection and co-cultivation of hypocotyls

*Agrobaterium* containing recombinant plasmids comprising over expressed SEQ ID NO: 1 and SEQ ID NOs: 19-24, respectively, were innoculated into LB medium containing 20 mg/L of Str, 50 mg/L of Kan and 40 mg/L of Rif and cultivated overnight at 28 °C, and bacteria were collected and resuspended with MS medium containing 100 mg/L acetosyringone till OD₆₀₀=0.4-0.6, followed by incubating at 28 °C for 1-2 h.

Pre-cultured healthy *Brassica napus* hypocotyls were immersed into bacteria liquid of *Agrobaterium* containing recombinant plasmids comprising over expressed SEQ ID NO: 1 and SEQ ID NOs: 19-24, respectively, for 30 s - 1 min, with constant oscillation to fully contact the bacteria liquid with hypocotyls. Spare bacteria liquid was fastly removed with aseptic filter paper. Then *brassica napus* hypocotyls were layed flat onto co-culture medium (MS+2 mg/L of 6-BA, 1 mg/L of 2,4-D, 2.5 mg/L of AgNO₃ and 19.62 mg/L of acetosyringone) to be co-cultivated for 2 days.

### 1-4. Screening culture and germ induction

The seven of co-cultivated *Brassica napus* hypocotyls were respectively innoculated into differential medium (MS+2 mg/L of 6-BA, 1 mg/L of 2,4-D, 2.5 mg/L of AgNO₃ and 19.62 mg/L of acetosyringone) to continue the cultivation. Germ callus was obtained after 4 weeks of cultivation with the medium refreshed by every 2 weeks.

### 1-5. Radication

The germ was sliced from callus tissue and transferred onto radication medium (1/2 MS, 0.15 mg/L of NAA and 250 mg/L of Cef) after the germ callus had grown up with 4-6 pieces of euphylla on screening culture medium (MS+2 mg/L of 6-BA, 2.5 mg/L of AgNO₃, 500 mg/L of carbenicillin and 10 mg/L of Kan). The culture tank was moved outdoors for 2-3 d after the root system of regenerated seedlings had grown well, followed by opening the tank and hardening seedlings for 2-3 d.

### 1-6. Pot culture

Transgenic plants comprising over expressed SEQ ID NO: 1 and SEQ ID NOs: 19-24 were cultivated respectively on radication medium to develop the entire root system, and then transferred to pot culture.

### 2. PCR detection of transgenic Brassica napus

Total DNA was extracted respectively from small amount of leaves of the regenerated plants that had grown well in soil, and PCR detection was performed with the DNA extracted as templates.
Primers for AtTR1 detection:
   Upstream primer (SEQ ID NO: 25): 5'-TGATCATTTGAGTTTATGTACCGA-3'
   Downstream primer (SEQ ID NO: 26): 5'-TCAAACTGGTGTTGGGACATTGGAT-3'
Primers for MT 1 detection:
   Upstream primer (SEQ ID NO: 27): 5'-AATCTGTTGAGAGTCGTATTTGCCAA-3'
   Downstream primer (SEQ ID NO: 28): 5'-TCAAACTGGTGTTGGGACATTGGAT-3'
Primers for MT2 detection:
   Upstream primer (SEQ ID NO: 29): 5'-GTCGTATTGGCCAAGAGGAAGATAG-3'
   Downstream primer (SEQ ID NO: 30): 5'-TCAAACTGGTGTTGGGACATTGGAT-3'
Primers for MT3 detection:
   Upstream primer (SEQ ID NO: 31): 5'-CTTGAAGCTCCTAGTGCTTGTAAT-3'
   Downstream primer (SEQ ID NO: 32): 5'-TCAAACTGGTGTTGGGACATTGGAT-3'
Primers for MT4 detection:
   Upstream primer (SEQ ID NO: 33): 5'-CTTGTGCTCGTAATGGTAGTTTAAAGT-3'
   Downstream primer (SEQ ID NO: 34): 5'-TCAAACTGGTGTTGGGACATTGGAT-3'
Primers for MT5 detection:
   Upstream primer (SEQ ID NO: 35): 5'-AAGTATGCTAACCGCAAGTGTGTTCT-3'
   Downstream primer (SEQ ID NO: 36): 5'-TCAAACTGGTGTTGGGACATTGGAT-3'
Primers for MT6 detection:
   Upstream primer (SEQ ID NO: 37): 5'-TATGCTCACCGCAAGTATGTTCAGCGTTT-3'
   Downstream primer (SEQ ID NO: 38): 5'-TCAGACTGGTGTTGGGTTGGATAT-3'

Then agarose electrophoresis was performed to detect the presence of target band (with a size of about 860bp), which was an indication that the target gene had been transformed into *Brassica napus.* And after such detections, brassica transformed with relevant genes were obtained.

### 3. Characterization of heat resisting capacity of transgenic brassica - seed germinations under heat shock

### 3-1. Seeds vernalization

7 kinds of transgenic *Brassica napus* seeds (AtTR1, MT1, MT2, MT3, MT4, MT5, and MT6) and non-transgenic wild type (WT) were selected, 200 for each, and incubated overnight in water at 4 °C.

### 3-2. Heat shock treatment

8 kinds of brassica seeds were respectively loaded into labeled tubes, 2 tubes for each kind, 100 seeds for each tube, and divided into groups A and B, wherein group A was incubated in water at 45 °C for 3 hours, and group B at room temperature for 2 hours.

### 3-3. Results

After 2 hours, seeds were placed in plates covered with filter paper and cultured in a chamber (illumination of 6,000-8,000 lux, 16h/8h light-dark cycles), and observed every 24 hours for the statistics of relative germination rates (relative germination rate = germination rate after heat shock / germination rate under room temperature × 100%), see Table 5.

**Table 5 Relative germination rates of brassica seeds after heat shock (%)**

| | AtTR1 | MT1 | MT2 | MT3 | MT4 | MT5 | MT6 | WT |
|---|---|---|---|---|---|---|---|---|
| 1 day later | 0 | 0 | 0 | 0 | 0 | 0 | 0 | 0 |
| 2 days later | 32.48 | 20.00 | 14.68 | 13.75 | 10.06 | 10.11 | 16.94 | 12.82 |
| 3 days later | 68.52 | 41.97 | 38.55 | 35.69 | 30.17 | 26.96 | 40.00 | 32.22 |
| 4 days later | 74.13 | 57.43 | 41.26 | 44.20 | 42.88 | 40.25 | 47.86 | 40.85 |
| 5 days later | 84.00 | 62.35 | 47.87 | 50.00 | 51.25 | 47.01 | 49.96 | 46.87 |
| 6 days later | 86.17 | 69.02 | 56.95 | 62.75 | 60.77 | 59.20 | 62.45 | 59.50 |
| 7 days later | 93.55 | 70.38 | 65.82 | 70.13 | 69.00 | 62.85 | 68.29 | 64.00 |

### 4. Characterization of transgenic brassica heat resisting capacity -growth status at the seedling stage at heat shock

7 kinds of transgenic *Brassica napus* seeds (AtTR1, MT1, MT2, MT3, MT4, MT5, and MT6) and non-transgenic wild type (WT) were respectively placed on moist filter papers for germination. After shell breaking, they were transferred into humus soil and cultured at 22°C for about 15 days (with the growth of two pieces of euphylla), then transfered into heat stress of 34°C under conditions of 14 h in sunlight and 10 h in dark. Growth status of each brassica was observed.

Results showed that, after 3 days of heat stress, the growth of *Brassica napus* was inhibited, wherein the transgenic MT1-6 and wild type all exhibited yellow and withered leaves, wherein MT1, MT2 and MT6 exhibited less yellowing and witheredness, and WT, MT3, MT4 and MT5 were relatively more severe, while AtTR1 grew normally. After 5 days of heat stress, MT1-6 and the wild type were all dead, while AtTR1 survived and grew normally.

### Example 5 Obtaining of the RcTR1 gene, a homologous gene of AtTR1 from castor oil plant, and heat resistance experiment of E. coli transformed with RcTR1

### 1. Cloning and obtaining of homologous gene from castor oil plant

The castor oil plant total RNA was extracted using TRIzol method (Invitrogen Corp., TRIzol agent) and reverse transcripted into cDNA (promega Corp., Reverse transcription kit). Primers were designed according to the RcTR1 nucleotide sequence from castor oil plant shown in SEQ ID NO: 39 (the amino acid sequence encoded by which is shown in SEQ ID NO: 40):
Upstream primer (SEQ ID NO: 41): 5'-GTGGTGTAGCAGGATTTTTAATC-3'
Downstream primer (SEQ ID NO: 42): 5'-GCATCCCCATTCATTTCAT-3'

Nucleotides 37-964 from SEQ ID NO: 39 were amplified from castor oil plant cDNA. The PCR product was purified (see manuals from Qiagen corp.) and sequenced to obtain the desired sequence.

2. Primers with restriction enzyme cutting site were designed according to the forementioned amplified sequence fragments, and the recombinant plasmid was constructed.
Upstream primer (SEQ ID NO: 43): 5'-CCGGAATTCATGAGTGATCAACTAGTTTTG-3'
Downstream primer (SEQ ID NO: 44): 5'-CCCAAGCTTTCATTGAAGTGGCTCTTG-3'

Nucleotides 68-946 from SEQ ID NO: 39 were amplified from the PCR product of step 1 (the amino acid sequence encoded by which is shown in SEQ ID NO: 40). The PCR product was purified (see manuals from Qiagen corp.) and digested with EcoR1 and HindIII. After being recovered from gels, fragments were respectively ligated into vector pET28 to obtain recombinant plasmids. The recombinant plasmids were transformed into E. coli JM109 and plated onto LB agar containing kanamycin, and sequenced to obtain E. coli JM109 strain containing recombinant plasmid.

3. After the E. coli JM109 strain was cultured, plasmid was extracted (see manuals from Qiagen corp.) and transformed into E. coli BL21 Rosset cells and plated onto LB agar containing kanamycin and chloramphenicol. Single colonies were picked and innoculated into LB medium containing 100 mg/L of kanamycin and chloramphenicol, and cultured overnight at 37°C and 225rpm. After cultivated, the culture was innoculated by 1: 250 into fresh LB medium (containing 100 mg/L of kanamycin and chloramphenicol) and OD600 value was detected (TU-1800 ultraviolet spectrophotometer, Puxitong instrument corp., Beijing, China). Different cultures were adjusted to the same OD value, and then subjected to shaking culture (37°C, 225rpm) for 3h, followed by the addition of 1 mM of IPTG, OD detection, and shaking culture again (42°C, 225rpm, 10h), wherein the OD value was detected every hour.

Results showed that, E. coli transformed with castor oil plant homologous gene RcTR1 exhibited higher heat resistance than non-transgenic E. coli. As was shown in Fig. 2, higher OD values stood for higher concentration of bacteria, i.e., better growth status. Varied with time, the growth curve slope of E. coli transformed with castor oil plant homologous gene (RcTR1) was significantly higher than that of non-transgenic E. coli (control), indicating that the growth rate and status of E. coli transformed with castor oil plant homologous gene RcTR1 was significantly better than that of non-transgenic E. coli.

### Example 6 Obtaining of genes OsTR1-1 and OsTR1-2, homologous genes of AtTR1 from rice, and heat resistance experiment of E. coli transformed with OsTR1-1 and OsTR1-2

### 1. Cloning and obtaining of homologous genes OsTR1-1 and OsTR1-2 from rice

The rice total RNA was extracted using TRIzol method (Invitrogen Corp., TRIzol agent) and reverse transcripted into cDNA (promega Corp., Reverse transcription kit). Primers were designed respectively according to nucleotide sequences shown in SEQ ID NO: 45 (the amino acid sequence encoded by which is shown in SEQ ID NO: 46) and SEQ ID NO: 47 (the amino acid sequence encoded by which is shown in SEQ ID NO: 48):
Primers for the amplification of OsTR1-1 sequence (SEQ ID NO: 45):
Upstream primer (SEQ ID NO: 49): 5'-AAACTTTTTTGGGTGATTTGC-3'
Downstream primer (SEQ ID NO: 50): 5'-CAAATTCTATTGCCCTTGTTCT-3'

Primers for the amplification of OsTR1-2 sequence (SEQ ID NO: 57):
Upstream primer (SEQ ID NO: 51): 5'-GGAATTTGGGATGGGCGACCA-3'
Downstream primer (SEQ ID NO: 52): 5'-AGAGACGCTACTGAAGTAACTAGCTAT-3'

Nucleotides 71-854 from OsTR1-1 sequence (SEQ ID NO: 45) (the amino acid sequence encoded by which is shown in SEQ ID NO: 46), and nucleotides 281-1150 from OsTR1-2 sequence (SEQ ID NO: 47) (the amino acid sequence encoded by which is shown in SEQ ID NO: 48) were respectively amplified from rice cDNA. The PCR product was purified (see manuals from Qiagen corp.) and sequenced to obtain the desired sequence.

2. Primers with restriction enzyme cutting sites were designed according to the forementioned amplified sequence fragments, and the recombinant plasmid was constructed.

Primers for OsTR1-1 sequence (SEQ ID NO: 45) containing restriction enzyme cutting sites:
Upstream primer (SEQ ID NO: 53): 5'-CGCGGATCCATGGGGGATCATGTTGCGGTGGAT-3'
Downstream primer (SEQ ID NO: 54): 5'-CCGGAGCTCCTATTGCCCTTGTTCTGGATGAGGT-3'

Primers for OsTR1-2 sequence (SEQ ID NO: 47) containing restriction enzyme cutting sites:
Upstream primer (SEQ ID NO: 55): 5'-CGCGGATCCATGGGCGACCATGTGGTGGT-3'
Downstream primer (SEQ ID NO: 56): 5'-CCGGAGCTCGACGCTACTGAAGTAACTAGCTAT-3'

Expression sequences were amplified from the PCR product of step 1 (sites 96-848 in SEQ ID NO: 45, sites 291-1147 in SEQ ID NO: 47). The PCR product was purified (see manuals from Qiagen corp.) and digested with BamH1 and Xhol1. After being recovered from gels, fragments were respectively ligated into vector pET28 to obtain recombinant plasmids. The recombinant plasmids were transformed into E. coli JM109 and plated onto LB agar containing kanamycin, and sequenced to obtain E. coli JM109 strain containing recombinant plasmid.

3. After the E. coli JM109 strain was cultured, plasmid was extracted (see manuals from Qiagen corp.) and transformed into E. coli BL21 Rosset cells and plated onto LB agar containing kanamycin and chloramphenicol. Single colonies were picked and innoculated into LB medium containing 100 mg/L of kanamycin and chloramphenicol, and cultured overnight at 37°C and 225rpm. After cultivated, the culture was innoculated by 1: 250 into fresh LB medium (containing 100 mg/L of kanamycin and chloramphenicol) and OD600 value was detected (TU-1800 ultraviolet spectrophotometer, Puxitong instrument corp., Beijing, China). Different cultures were adjusted to the same OD value, and then subjected to shaking culture (37°C, 225rpm) for 3h, followed by the addition of 1 mM of IPTG, OD detection, and shaking culture again (42 °C, 225rpm, 10h), wherein the OD value was detected every hour.

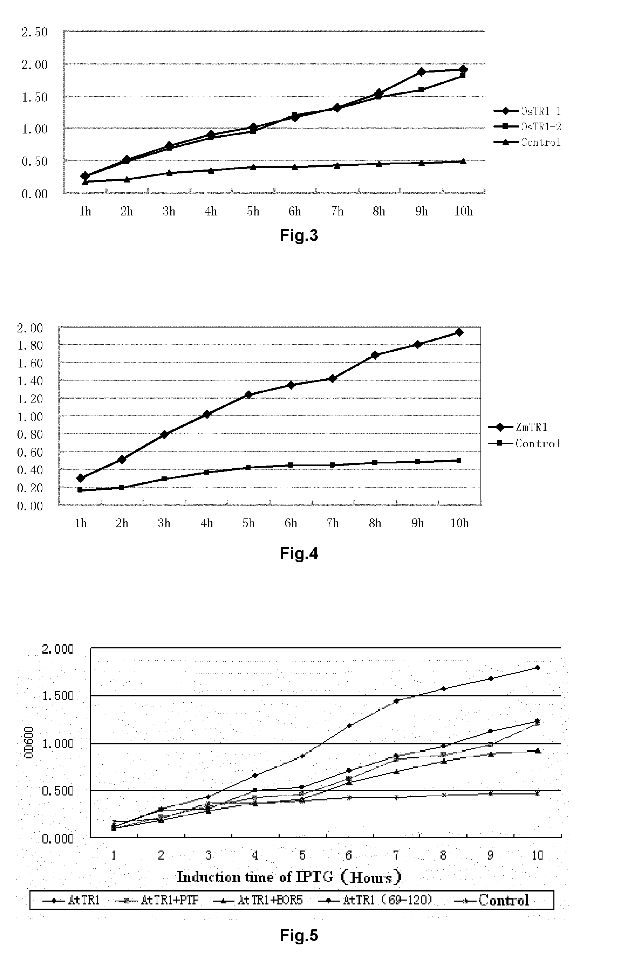

Results showed that, E. coli transformed with the two rice homologous genes OsTR1-1 and OsTR1-2 exhibited higher heat resistance than non-transgenic E. coli. As was shown in Fig. 3, higher OD values stood for higher concentration of bacteria, i.e., better growth status. Varied with time, the growth curve slope of E. coli transformed with rice homologous gene (OsTR1-1 and OsTR1-2) was significantly higher than that of non-transgenic E. coli (control), indicating that the growth rate and status of E. coli transformed with rice homologous gene OsTR1-1 and OsTR1-2 was significantly better than that of non-transgenic E. coli.

### Example 7 Obtaining of the ZmTR1 gene, a homologous gene of AtTR1 from corn, and heat resistance experiment of E. coli transformed with ZmTR1

### 1. Cloning and obtaining of corn homologous gene ZmTR1

The corn total RNA was extracted using TRIzol method (Invitrogen Corp., TRIzol agent) and reverse transcripted into cDNA (promega Corp., Reverse transcription kit). Primers were designed according to SEQ ID NO: 57 from corn (the amino acid sequence encoded by which is shown in SEQ ID NO: 58):
Upstream primer (SEQ ID NO: 59): 5'-TTGGGATGGCTGGTGACG-3'
Downstream primer (SEQ ID NO: 60): 5'-GTGAGCAACTACTGGGGATGTG-3'

Nucleotides 153-1051 from SEQ ID NO: 54 were amplified from corn cDNA. The PCR product was purified (see manuals from Qiagen corp.) and sequenced to obtain the desired sequence.

2. Primers with restriction enzyme cutting sites were designed according to the forementioned amplified sequence fragments, and the recombinant plasmid was constructed.
Upstream primer (SEQ ID NO: 61): 5'-CCGGAATTCATGGCTGGTGACGACC-3'
Downstream primer (SEQ ID NO: 62): 5'-CCCAAGCTTCTATTGCTGTTGCTGCGAC-3'

Expression sequences were amplified from the PCR product of step 1 (sites 158-1018 in SEQ ID NO: 54, the amino acid sequence encoded by which is shown in SEQ ID NO: 58). The PCR product was purified (see manuals from Qiagen corp.) and digested with EcoR1 and HindIII. After being recovered from gels, fragments were respectively ligated into vector pET28 to obtain recombinant plasmids. The recombinant plasmids were transformed into E. coli JM109 and plated onto LB agar containing kanamycin, and sequenced to obtain E. coli JM109 strain containing recombinant plasmid.

3. After the E. coli JM109 strain was cultured, plasmid was extracted (see manuals from Qiagen corp.) and transformed into E. coli BL21 Rosset cells and plated onto LB agar containing kanamycin and chloramphenicol. Single colonies were picked and innoculated into LB medium containing 100 mg/L of kanamycin and chloramphenicol, and cultured overnight at 37°C and 225rpm. After cultivated, the culture was innoculated by 1: 250 into fresh LB medium (containing 100 mg/L of kanamycin and chloramphenicol) and OD600 value was detected (TU-1800 ultraviolet spectrophotometer, Puxitong instrument corp., Beijing, China). Different cultures were adjusted to the same OD value, and then subjected to shaking culture (37°C, 225rpm) for 3h, followed by the addition of 1 mM of IPTG, OD detection, and shaking culture again (42 °C, 225rpm, 10h), wherein the OD value was detected every hour.

Results showed that, E. coli transformed with corn homologous gene ZmTR1 exhibited higher heat resistance than non-transgenic E. coli. As was shown in Fig. 4, higher OD values stood for higher concentration of bacteria, i.e., better growth status. Varied with time, the growth curve slope of E. coli transformed with corn homologous gene (ZmTR1) was significantly higher than that of non-transgenic E. coli (control), indicating that the growth rate and status of E. coli transformed with corn homologous gene ZmTR1 was significantly better than that of non-transgenic E. coli.

### Example 8 Studies on the AtTR1 structure

In this example, PTP and BOR5 transmembrane domains were used as a substitution of AtTR1 intrinsic transmembrane domain (amino acids 180-239 in SEQ ID NO.2), and AtTR1 intrinsic transmembrane domain was deleted; in order to study the heat resistance of AtTR1, AtTR1 with substituted transmembrane domain, and transmembrane-domain-deleted AtTR1 comprising the RINGv structure only (sites 69-120 in SEQ ID NO.2, the amino acid sequence of which was shown in SEQ ID NO.63).

### 1. Amplification of base sequences encoding protein fragments

Since the forementioned two transmembrane domains were both derived from arabidopsis, the arabidopsis cDNA was used as a template, and base sequences encoding two transmembrane domains were amplified by PCR, respectively. Meanwhile, base sequences encoding amino acids 1-179 and 69-120 in SEQ ID NO.2 were also amplified by PCR from the arabidopsis cDNA.

### 2. Vector construction the obtaining of recombinant bacteria

The forementioned amplified gene fragments as well as the pET28a vector was respectively digested with EcoRI and Ecl136II, recovered from the gel, and ligated and transformed into E. coli DH5a. Positive clones were picked and plasmids were extracted, transformed into E. coli Rosseta cells and plated onto LB agar containing kanamycin and chloramphenicol. Single colonies were picked and 4 recombinant bacteria were obtained as follows:
Recombinant bacteria comprising the entire AtTR1 protein, named AtTR1;
Recombinant bacteria with AtTR1 intrinsic transmembrane domain substituted by PTP transmembrane domain, named AtTR1+PTP;
Recombinant bacteria with AtTR1 intrinsic transmembrane domain substituted by BOR5 transmembrane domain, named AtTR1+BOR5;
Recombinant bacteria comprising AtTR1 (amino acids 69-120), i.e., comprising the RINGv structure only and without transmembrane domain, named AtTR1 (69-120).

### 3. Detection of heat resistance

Single colonies were picked and innoculated into LB medium containing 50 mg/L of kanamycin and 34 mg/L of chloramphenicol, and cultured overnight at 37°C and 225rpm. After cultivated, the culture was innoculated by 1: 100 into fresh LB medium (containing 50 mg/L of kanamycin and 34 mg/L of chloramphenicol) and cultivated at 37°C and 225rmp for 2 hours, and OD600 value was detected (TU-1800 ultraviolet spectrophotometer, Puxitong instrument corp., Beijing, China) till about 0.2. IPTG was then added to a final concentration of 1mM and cultivation was continued at 42°C and 225rpm with shaking, wherein the OD value was detected every hour, and the growth curve was made according to OD values. The growth curve was shown in Table 6 and Fig. 5.

**Table 6 Growth curves (OD600 values)**

| IPTG induction times | AtTR1 | AtTR1 +PTP | AtTR1 +BOR5 | AtTR1 (69-120) | Blank vector control |
|---|---|---|---|---|---|
| 1 hour | 0.115 | 0.102 | 0.100 | 0.114 | 0.167 |
| 2 hours | 0.300 | 0.217 | 0.184 | 0.293 | 0.199 |
| 3 hours | 0.429 | 0.329 | 0.291 | 0.300 | 0.363 |
| 4 hours | 0.659 | 0.420 | 0.366 | 0.495 | 0.366 |
| 5 hours | 0.863 | 0.459 | 0.401 | 0.529 | 0.387 |
| 6 hours | 1.185 | 0.622 | 0.585 | 0.711 | 0.421 |
| 7 hours | 1.441 | 0.824 | 0.700 | 0.862 | 0.426 |
| 8 hours | 1.566 | 0.866 | 0.814 | 0.963 | 0.449 |
| 9 hours | 1.680 | 0.975 | 0.885 | 1.126 | 0.461 |
| 10 hours | 1.796 | 1.208 | 0.923 | 1.233 | 0.460 |

As was shown in the result, groups expressing the entire AtTR1 protein exhibited the best heat resistance, significantly higher than those of AtTR1+PTP, AtTR1+BOR5, AtTR1 (69-120) and blank vector control. Groups expressing substituted transmembrane domains, namely AtTR1+TP, AtTR1+BOR5 and AtTR1 (69-120), were possessed with some heat resistance, though significantly lower than that of AtTR1 group. The blank vector control exhibited the worst heat resistance and could hardly grow under the high temperature of 42°C.

### Example 9 Studies on the structure of OsTR1-1, the rice homologous protein of AtTR1

In this example, PTP and BOR5 transmembrane domains were used as a substitution of OsTR1-1 intrinsic transmembrane domain (amino acids 145-203 of the protein synthesized by SEQ ID NO.45), and OsTR1-1 intrinsic transmembrane domain was deleted; in order to study heat resistance of OsTR1-1, OsTR1-1 with substituted transmembrane domain, and transmembrane-domain-deleted OsTR1-1 comprising the RINGv structure only (amino acids 32-81 of the protein synthesized by SEQ ID NO.45, the amino acid sequence of which was shown in SEQ ID NO.66).

The experiment procedure was the same as Example 8, and 4 recombinant bacteria were obtained, as follows:
Recombinant bacteria comprising the entire OsTR1-1 protein, named OsTR1-1;
Recombinant bacteria with OsTR1-1 intrinsic transmembrane domain substituted by PTP transmembrane domain, named OsTR1-1+PTP;
Recombinant bacteria with OsTR1-1 intrinsic transmembrane domain substituted by BOR5 transmembrane domain, named OsTR1-1+BOR5;
Recombinant bacteria comprising OsTR1-1 (amino acids 32-81), i.e., comprising the RINGv structure only and without transmembrane domain, named OsTR1-1 (32-81).

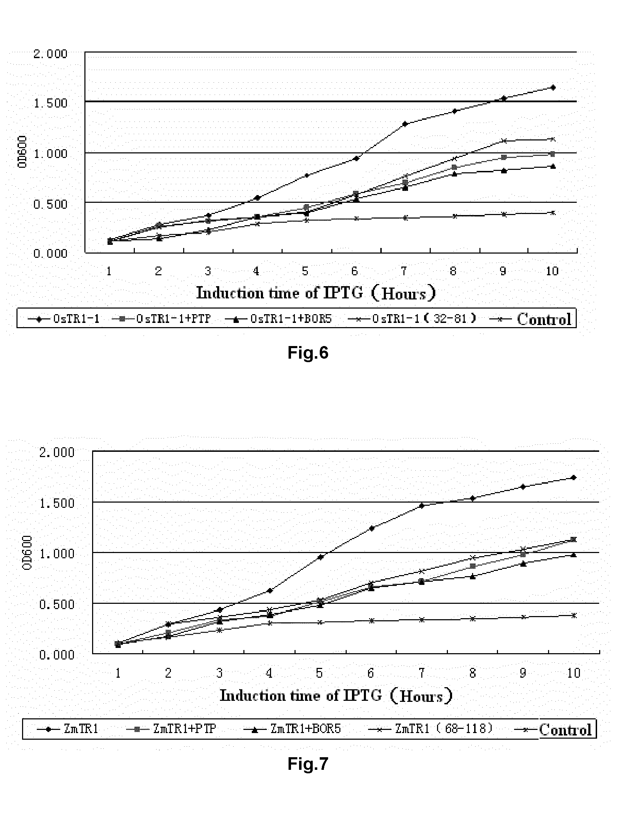

After shaking cultivated with 1mM of IPTG at 42°C and 225rpm for 10 hours, the growth curve was shown in Table 7 and Fig. 6.

**Table 7 Growth curves (OD600 values)**

| IPTG inducing times | OsTR1-1 | OsTR1-1+PTP | OsTR1-1+BOR5 | OsTR1-1 (32-81) | Blank vector control |
|---|---|---|---|---|---|
| 1 hour | 0.127 | 0.110 | 0.109 | 0.110 | 0.106 |
| 2 hours | 0.275 | 0.263 | 0.136 | 0.251 | 0.168 |
| 3 hours | 0.369 | 0.312 | 0.231 | 0.318 | 0.204 |
| 4 hours | 0.538 | 0.356 | 0.351 | 0.357 | 0.289 |
| 5 hours | 0.768 | 0.449 | 0.399 | 0.403 | 0.317 |
| 6 hours | 0.933 | 0.584 | 0.531 | 0.575 | 0.338 |
| 7 hours | 1.280 | 0.694 | 0.649 | 0.759 | 0.345 |
| 8 hours | 1.411 | 0.841 | 0.782 | 0.938 | 0.359 |
| 9 hours | 1.538 | 0.949 | 0.821 | 1.114 | 0.381 |
| 10 hours | 1.645 | 0.981 | 0.860 | 1.129 | 0.394 |

As was shown in the result, groups expressing the entire OsTR1-1 protein exhibited the best heat resistance; significantly higher than those of OsTR1-1+PTP, OsTR1-1+BOR5, OsTR1-1 (32-81) and blank vector control. Groups expressing substituted transmembrane domains, namely OsTR1-1+PTP, OsTR1-1+BOR5 and OsTR1-1 (32-81), were possessed with some heat resistance, though significantly lower than that of OsTR1-1 group. The blank vector control exhibited the worst heat resistance and could hardly grow under the high temperature of 42°C.

### Example 10 Studies on the structure of ZmTR1, the corn homologous protein of AtTR1

In this example, PTP and BOR5 transmembrane domains were used as a substitution of ZmTR1 intrinsic transmembrane domain (amino acids 175-234 of the protein synthesized by SEQ ID NµO.57), and ZmTR1 intrinsic transmembrane domain was deleted; in order to study heat resistance of ZmTR1, ZmTR1 with substituted transmembrane domain, and transmembrane-domain-deleted ZmTR1 comprising the RINGv structure only (amino acids 68-118 of the protein synthesized by SEQ ID NO. 57, the amino acid sequence of which was shown in SEQ ID NO.65).

The experiment procedure was the same as Example 8, and 4 recombinant bacteria were obtained, as follows:
Recombinant bacteria comprising the entire ZmTR1 protein, named ZmTR1;
Recombinant bacteria with ZmTR1 intrinsic transmembrane domain substituted by PTP transmembrane domain, named ZmTR1+PTP;
Recombinant bacteria with ZmTR1 intrinsic transmembrane domain substituted by BOR5 transmembrane domain, named ZmTR1+BOR5;
Recombinant bacteria comprising ZmTR1 (amino acids 68-118), i.e., comprising the RINGv structure only and without transmembrane domain, named ZmTR1 (68-118).

After shaking cultivated with 1mM of IPTG at 42°C and 225rpm for 10 hours, the growth curve was shown in Table 8 and Fig. 7.

**Table 8 Growth curves (OD600 values)**

| IPTG inducing times | ZmTR1 | ZmTR1 +PTP | ZmTR1 +BOR5 | ZmTR1 (68-118) | Blank vector control |
|---|---|---|---|---|---|
| 1 hour | 0.108 | 0.100 | 0.100 | 0.106 | 0.102 |
| 2 hours | 0.300 | 0.208 | 0.177 | 0.294 | 0.161 |
| 3 hours | 0.436 | 0.341 | 0.319 | 0.368 | 0.239 |
| 4 hours | 0.622 | 0.372 | 0.389 | 0.439 | 0.305 |
| 5 hours | 0.955 | 0.513 | 0.481 | 0.527 | 0.317 |
| 6 hours | 1.243 | 0.665 | 0.653 | 0.706 | 0.328 |
| 7 hours | 1.458 | 0.711 | 0.714 | 0.814 | 0.339 |
| 8 hours | 1.542 | 0.865 | 0.765 | 0.949 | 0.352 |
| 9 hours | 1.656 | 0.981 | 0.894 | 1.034 | 0.362 |
| 10 hours | 1.741 | 1.118 | 0.986 | 1.131 | 0.380 |

As was shown in the result, groups expressing the entire ZmTR1 protein exhibited the best heat resistance; significantly higher than those of ZmTR1+TP, ZmTR1+BOR5, ZmTR1 (68-118) and blank vector control. Groups expressing substituted transmembrane domains, namely ZmTR1+PTP, ZmTR1+BOR5 and ZmTR1 (68-118), were possessed with some heat resistance, though significantly lower than that of ZmTR1 group. The blank vector control exhibited the worst heat resistance and could hardly grow under the high temperature of 42 °C.

### Example 11 Characterization of heat resistance of arabidopsis over expressed with AtTR1, OsTR1-2, ZmTR1 and RcTR1 genes and critical fragments thereof

Arabidopsis was transformed with AtTR1 gene (SEQ ID NO: 1), OsTR1-2 gene (SEQ ID NO: 47), ZmTR1 gene (SEQ ID NO: 57) and RcTR1 gene (SEQ ID NO: 39); and critical fragments thereof encoding peptides comprising SEQ ID NO: 63, SEQ ID NO: 67, SEQ ID NO: 65 and SEQ ID NO: 64, in order to study the heat resistance thereof.

Vectors were constructed according to the method in Example 2, and arabidopsis was transformed via in floral dip method, with steps as follows:
*1. Agrobaterium* containing over expression recombinant plasmids comprising SEQ ID NO: 1, SEQ ID NO: 47, SEQ ID NO: 57, SEQ ID NO: 39, SEQ ID NO: 63, SEQ ID NO: 67, SEQ ID NO: 65 and SEQ ID NO: 64 were innoculated into LB medium containing 20 mg/L of Str, 50 mg/L of Kan and 40 mg/L of Rif, and cultivated overnight at 28°C. Cells were collected and resuspended with MS medium containing 0.01% of surfactant silwet-77 till OD₆₀₀=0.4-0.6, followed by incubating at 28 °C for 1-2 h and keeping the bacteria liquid.
2. The inflorescence of *Arabidopsis thaliana* cultured for 60 days was cut off and immersed with the bacteria liquid of *Agrobaterium* containing recombinant plasmids for 2 min. After cultivating in dark for 48 hours, the result *Arabidopsis thaliana* seedlings were transferred in a normal illumination environment, with resulted legumes as the transgenic T0 generation of the seed.
3. The resulted seeds were planted and after 50 days of growth, positive plants were identified via PCR, as follows:
   Transgenic arabidopsis comprising AtTR1 (SEQ ID NO: 1), named AtTR1,
   Transgenic arabidopsis comprising AtTR1 critical peptides (SEQ ID NO: 63) encoding sequence, named R-AtTR1,
   Transgenic arabidopsis comprising OsTR1-2 (SEQ ID NO: 47), named At-OsTR1-2,
   Transgenic arabidopsis comprising OsTR1-2 critical peptides (SEQ ID NO: 67) encoding sequence, named At-R-OsTR1-2,
   Transgenic arabidopsis comprising ZmTR1 (SEQ ID NO: 57), named At-ZmTR1,
   Transgenic arabidopsis comprising ZmTR1 critical peptides (SEQ ID NO: 65) encoding sequence, named At-R-ZmTR1,
   Transgenic arabidopsis comprising RcTR1 (SEQ ID NO: 39), named At-RcTR1,
   Transgenic arabidopsis comprising RcTR1 critical peptides (SEQ ID NO: 64) encoding sequence, named At-R-RcTR1,

After the maturation of transgenic plants, seeds were collected for reservation.

### 4. Germination of seeds under heat stress

MS culture medium preparations (see Table 8, with pH adjusted to 5.8 with KOH) were separately loaded into plates after being sterilized with high-pressure steam. After solidified, the culture medium was innoculated with 2 ml of seeds suspended in sterile water, the spare of which was removed after seeding well. The plate cover was opened to dry the medium surface at sterile environment for 1 h. The plate was sealed.

The experiment was divided into two groups. The first group served as the heat shock group, wherein the plate was cultivated in a culture chamber (50°C, illumination intensity 6,000-8,000 lx, 16 h/8h light-dark cycles, relative humidity 70%) to be treated with heat shock for 2 hours, then subjected to room temperature (22°C; intensity of illumination, 6,000 - 8,000 lx; light-dark cycles, 16h/8h; relative humidity 70%). Germination and other phenotypes were observed every day for the statistics of germination number. And the second group was a non-heat shock control, wherein the plate was cultivated in a culture chamber (22°C, illumination intensity 6,000-8,000 lx, 16h/8h light-dark cycles, relative humidity 70%). Germination and other phenotypes were observed every day for the statistics of germination number, see Table 9.

**Table 8 Components of MS culture medium**

| Components | | Conc. (mg/L) |
|---|---|---|
| Major elements | Potassium nitrate (KNO₃) | 1900 |
| | Ammonium nitrate (NH₄NO₃) | 1650 |
| | Potassium dihydrogen phosphate (KH₂PO₄) | 170 |
| | Magnesium sulfate (MgSO₄·7H₂O) | 370 |
| | Calcium chloride (CaCl₂·2H₂O) | 440 |
| Minor elements | Potassium iodide (KI) | 0.83 |
| | Boric acid (H₃BO₃) | 6.2 |
| | Manganese sulfate (MnSO₄·4H₂O) | 22.3 |
| | Zinc sulfate (ZnSO₄·7H₂O) | 8.6 |
| | Sodium molybdate (Na₂MoO₄·2H₂O) | 0.25 |
| | Copper sulfate (CuSO₄·5H₂O) | 0.025 |
| | Cobalt chloride (CoCl₂·6H₂O) | 0.025 |
| Ferric salts | Disodium ethylenediamine tetraacetic acid | 37.3 |
| | Ferrous sulfate (FeSO₄·7H₂O) | 27.8 |
| Organic components | Inositol | 100 |
| | Glycine | 2 |
| | Thiamine hydrochloride (VB1) | 0.1 |
| | Pyridoxine hydrochloride (VB6) | 0.5 |
| | Nicotinic acid (VB5 or VPP) | 0.5 |
| | Sucrose | 30g/L |
| | Agar | 7g/L |

**Table 9 Germination rates of arabidopsis seeds after heat shock**

| | Day 5 at 22 °C after heat shock in 50 °C | Day 5 without heat shock |
|---|---|---|
| AtTR1 | 74% | 100% |
| R-AtTR1 | 62% | 100% |
| At-OsTR 1-2 | 56% | 96% |
| At-R-OsT R1-2 | 40% | 98% |
| At-ZmTR 1 | 60% | 100% |
| At-R-Zm TR1 | 42% | 98% |
| At-RcTR 1 | 48% | 100% |
| At-R-RcT R1 | 30% | 100% |
| WT | 6% | 98% |

Results showed that, the germination rates of arabidopsis seeds were affected after heat shock treatment, and either the transgenic type or non-transgenic control exhibited lower germination rate after heat shock than those under room temperature. Comparisons indicated that, transgenic arabidopsis AtTR1, At-OsTR1-2, At-ZmTR1 and At-RcTR1, which were transformed with the entire AtTR1, OsTR1-2, ZmTR1 and RcTR1 genes, were affected the least by heat shocks with germination rates of 74%-48%. Transgenic arabidopsis R-AtTR1, At-R-OsTR1-2, At-R-ZmTR1 and At-R-RcTR1, which were transformed with the critical sequences of AtTR1, OsTR1-2, ZmTR1 and RcTR1, were affected less by heat shocks (better than non-transgenic) with germination rates of 62%-30%. While the germination rate of the non-transgenic was merely 6%.

Arabidopsis after heat shock treatment was continued to be cultivated under conditions of 22°C, illumination intensity 6,000-8,000 1x, 16h/8h light-dark cycles, and relative humidity of 70%. Results showed that, as time passed, the non-transgenic control grew no green base leaves, with yellow or white seedlings and finaly died; while transgenic ones could exhibit green base leaves with good growth status, and could blossom and breed.

Results indicated that, genes of AtTR1, OsTR1-2, ZmTR1 and RcTR1, either entire sequences (SEQ ID NO: 1, SEQ ID NO: 47, SEQ ID NO: 57 and SEQ ID NO: 39) or critical sequences (SEQ ID NO: 63, SEQ ID NO: 67, SEQ ID NO: 65 and SEQ ID NO: 64), could all enhance heat resistance of plants, leading to good germination and growth.

### Example 12 E3 ubiquitin ligase activity of proteins AtTR1, OsTR1-1 and ZmTR1

20ul of reaction systems were prepared, comprising: 1ug E1 protein, 2ug E2 protein, 4ug AtTR1 protein (or OsTR1-1 protein, or ZmTR1 protein), 0.1 mmol/L of ubiquitin (Ub), 2 mmol/L of ATP, 5 mmol/L of MgCl₂, and 50 mmol/L of Tris at pH7.5. Reaction was performed at 30°C for 90min, and then 6×SDS-PAGE loading buffer was added to terminate the reaction. After boiling for 5min, SDS-PAGE electrophoresis was performed.

After electrophoresis, the pruduct was electro-transferred to polyvinylidene difluoride (PVDF) membrane, and Western Blot detection was performed with anti-Ub monoclonal antibody (purchased from Santa Cruz) and alkaline phosphatase-conjugated secondary rat antibody (AP, purchased from Promega).

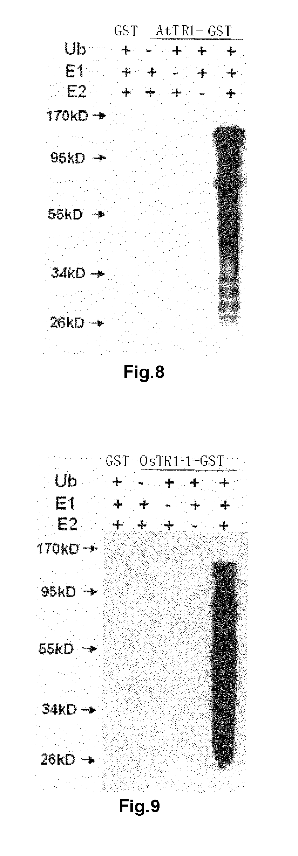

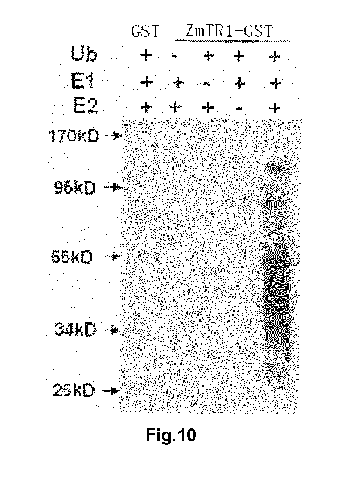

As shown in Fig. 8, 9 and 10, with the co-functioning of proper E1, E2, ATP and Ub in protein reactions *in vitro,* Ub could be conjugated with proteins AtTR1, OsTR1-1 or ZmTR1 and form poly-chains. As was shown, multiple bands occurred in the lane with the addition of E1, E2, ATP and Ub, while no band occurred in the control, indicating that proteins AtTR1, OsTR1-1 and ZmTR1 were possessed with ubiquitin E3 ligase activity and could catalyze the formation of monomer or poly-ubiquitins.

As was shown in the forementioned examples, protein AtTR1 of the invention and homologous proteins thereof in plants such as corn, rice and castor oil plant showed capability of enhancing heat resistance of plants or microorganisms. Research showed that, such kind of proteins all comprised zinc finger of RINGv type, and belonged to E3 ligase. The invention had also found the critical region of the protein - RINGv zinc finger, wherein E3 ligase proteins comprising such a structure would enhance heat resistance of plants or microorganisms, while proteins being deleted with the structure would totally lose the function. In addition, the transmembrane domain was studied for the protein, and results showed that proteins comprising the intrinsic transmembrane domain could better enhance heat resistance of plants or microorganisms, and proteins with transmembrane domain substituted by others could still exhibit some heat resistance, better than that of non-transgenic type. To sum up, RINGv zinc finger was the critical peptide structure of AtTR1 proteins that led to the heat resistance, said structure comprising N'- CRICQE X₇₋₄₅ PCAC X₆ AHR X₁ CVQ X₁₃₋₂₇ -C' or further comprising N'- CRICQEED X₃₋₂₀ NL X₃₋₂₀ PCAC X₂ SLK X₁ AHR X₁ CVQRWC X₁₀₋₂₄ -C' (wherein X represents any amino acid and the subscript represents the number of amino acid). The invention thus provided new and promising alternative genes encoding heat-resisting proteins or peptides, and proteins or peptides encoded by the same, as well as uses of these genes, proteins and peptides for the enhancement of heat resistance of plants and microorganisms.

## Claims

1. A gene encoding protein able to enhance heat resistance of plants or microorganisms, **characterized in that** it comprises nucleotide sequence encoding peptides as follows:
N'- CRICQE X₇₋₄₅ PCAC X₆ AHR X₁ CVQ X₁₃₋₂₇ -C', wherein X represents any amino acid and the subscript represents the number of amino acid.

2. Said gene encoding protein able to enhance heat resistance of plants or microorganisms according to claim 1, **characterized in that** said peptides are: N'- CRICQEED X₃₋₂₀ NL X₃₋₂₀ PCAC X₂ SLK X₁ AHR X₁ CVQRWC X₁₀₋₂₄ -C', wherein X represents any amino acid and the subscript represents the number of amino acid.

3. Said gene encoding protein able to enhance heat resistance of plants or microorganisms according to claim 1, **characterized in that** said protein is an ubiquitin ligase.

4. Said gene encoding protein able to enhance heat resistance of plants or microorganisms according to claim 1, **characterized in that** said protein is a transmembrane protein.

5. Said gene encoding protein able to enhance heat resistance of plants or microorganisms according to claim 1, **characterized in that** said protein further comprises transmembrane domains.

6. Said gene encoding protein able to enhance heat resistance of plants or microorganisms according to claim 4, **characterized in that** said protein further comprises 1-6 transmembrane domains.

7. Said gene encoding protein able to enhance heat resistance of plants or microorganisms according to claim 4, **characterized in that** said protein further comprises 2-3 transmembrane domains.

8. Said gene encoding protein able to enhance heat resistance of plants or microorganisms according to claim 7, **characterized in that** said transmembrane domains comprise at least one structure shown in N'- A X₂₋₆ CRS X₂₋₈ LIL X₂₋₄ LL X₁₋₄ LR X₁₋₁₀ -C' or N'- L X₂₋₄ R X₁₋₅ GFLL X₁₋₇ YIMAW X₁₋₁₅ -C', wherein X represents any amino acid and the subscript represents the number of amino acid.

9. Said gene encoding protein able to enhance heat resistance of plants or microorganisms according to claim 4, **characterized in that** said protein further comprises a signal peptide.

10. Said gene encoding protein able to enhance heat resistance of plants or microorganisms according to claim 9, **characterized in that** said signal peptide is a membrane localization signal peptide.

11. Said gene encoding protein able to enhance heat resistance of plants or microorganisms according to any one of claims 1-4, **characterized in that** said gene is derived from plant genome.

12. Said gene encoding protein able to enhance heat resistance of plants or microorganisms according to claim 5, **characterized in that** said plant is arabidopsis, rice, corn or castor oil plant.

13. Said gene encoding protein able to enhance heat resistance of plants or microorganisms according to any one of claims 1-12, **characterized in that** (1): said gene comprises nucleotide sequences shown in SEQ ID NO: 39, SEQ ID NO: 1, SEQ ID NO: 45, SEQ ID NO: 47 or SEQ ID NO: 57;
Or (2): said gene comprises nucleotide sequences derived from substitution, deletion or addition of at least one nucleotide in any one of said nucleotide sequences in (1), while encoding proteins with the same or similiar function.

14. Said gene encoding protein able to enhance heat resistance of plants or microorganisms according to claim 11, **characterized in that** (1): said gene comprises nucleotide sequences of sites 158-1018 in SEQ ID NO: 57, sites 96-848 in SEQ ID NO: 45, sites 291-1127 in SEQ ID NO: 47 or sites 68-946 in SEQ ID NO: 39;
Or (2): said gene comprises nucleotide sequences derived from substitution, deletion or addition of at least one nucleotide in any one of said nucleotide sequences in (1), while encoding proteins with the same or similiar function.

15. A protein able to enhance heat resistance of plants or microorganisms, **characterized in that** it comprise peptides as follows:
N'- CRICQE X₇₋₄₅ PCAC X₆ AHR X₁ CVQ X₁₃₋₂₇ -C', wherein X represents any amino acid and the subscript represents the number of amino acid.

16. Said protein able to enhance heat resistance of plants or microorganisms according to claim 15, **characterized in that** said peptides comprise structures of: N'- CRICQEED X₃₋₂ NL X₃₋₂₀ PCAC X₂ SLK X₁ AHR X₁ CVQRWC X₁₀₋₂₄ -C', wherein X represents any amino acid.

17. Said protein able to enhance heat resistance of plants or microorganisms according to claim 15, **characterized in that** said protein further comprises transmembrane domains.

18. Said protein able to enhance heat resistance of plants or microorganisms according to claim 17, **characterized in that** said protein further comprises 1-6 transmembrane domains.

19. Said protein able to enhance heat resistance of plants or microorganisms according to claim 18, **characterized in that** said protein comprises 2-3 transmembrane domains.

20. Said protein able to enhance heat resistance of plants or microorganisms according to claim 17, **characterized in that** said transmembrane domains comprise structures shown in at least one of N'- A X₂₋₆ CRS X₂₋₈ LIL X₂₋₄ LL X₁₋₄ LR X₁₋₁₀ -C' or N'- L X₂₋₄ R X₁₋₅ GFLL X₁₋₇ YIMAW X₁₋₁₅ -C', wherein X represents any amino acid and the subscript represents the number of amino acid.

21. Said protein able to enhance heat resistance of plants or microorganisms according to claim 15, **characterized in that** said protein is an ubiquitin ligase.

22. Said protein able to enhance heat resistance of plants or microorganisms according to claim 15, **characterized in that** said protein further comprises a signal peptide.

23. Said protein able to enhance heat resistance of plants or microorganisms according to claim 22, **characterized in that** said signal peptide is a membrane localization signal peptide.

24. Said protein able to enhance heat resistance of plants or microorganisms according to any one of claims 15-23, wherein said protein is derived from plants.

25. Said protein able to enhance heat resistance of plants or microorganisms according to any one of claims 15-24, **characterized in that** (1) said protein comprises amino acid sequences encoded by nucleotide sequences shown in SEQ ID NO: 1, sites 158-1018 in SEQ ID NO: 57, sites 96-848 in SEQ ID NO: 45, sites 291-1127 in SEQ ID NO: 47 or sites 68-946 in SEQ ID NO: 39;
Or (2): said protein comprises amino acid sequences derived from substitution, deletion or addition of at least one amino acid in any one of amino acid sequences in (1), while possessing with the same or similiar function.

26. A gene encoding said protein able to enhance heat resistance of plants or microorganisms according to any one of claims 15-25.

27. A peptide, **characterized in that** it comprises domains as shown in N'- CRICQE X₇₋₄₅ PCAC X₆ AHR X₁ CVQ X₁₃₋₂₇ -C', wherein X represents any amino acid and the subscript represents the number of amino acid.

28. Said peptide according to claim 27, **characterized in that** it comprises domains as shown in N'- CRICQEED X₃₋₂₀ NL X₃₋₂₀ PCAC X₂ SLK X₁ AHR X₁ CVQRWC X₁₀₋₂₄ -C', wherein X represents any amino acid and the subscript represents the number of amino acid.

29. Said peptide according to claim 27, **characterized in that** it is derived from plants.

30. Said peptide according to claim 27, **characterized in that** said plant is arabidopsis, rice, corn or castor oil plant.

31. Said peptide according to claims 27 or 28, **characterized in that** it can form a zinc finger.

32. Said peptide according to claims 27 or 28, **characterized in that** it enables proteins to enhance heat resistance of plants or microorganisms.

33. Said peptide according to any one of claims 27-32, **characterized in that** it comprises polypeptide sequences as follows:
(1): amino acid sequences shown in SEQ ID NO: 63, SEQ ID NO: 64, SEQ ID NO: 65, SEQ ID NO: 66 or SEQ ID NO: 67;
Or, amino acid sequences derived from substitution, deletion or addition of at least one amino acid in any one of polypeptides in (1), while possessing with the same or similiar function.

34. Nucleotide sequences encoding any one of said peptides in claims 27-33.

35. A method of modifying genes encoding non- heat-resisting proteins into those encoding heat-resisting ones, comprising the steps of inserting nucleotide sequences encoding peptide of any one of claims 27-33 into genes encoding non- heat-resisting proteins, or substituting one section of nucleotide sequences therein; wherein such an insertion or substitution occurs between codons, therefore the modified gene still has the ability to encode an entire protein.

36. Said method according to claim 35, **characterized in that** the nucleotide sequence substituted in said gene encoding non- heat-resisting protein is the nucleotide sequence encoding zinc finger domain.

37. Said method according to claims 35 or 36, **characterized in that** said non- heat-resisting protein is an ubiquitin ligase.

38. A method for enhancing plant heat resistance or preparing plants with high heat resistance, including steps of:
a. Operably linking said gene of any one of claims 1-14 with expression regulation sequence of a vector to form a recombinant vector expressing said gene;
b. Transforming the recombinant vector of step (1) into plant cells;
c. Selecting and obtaining transformed cells, followed by regenerating the transformed cells to form transgenic plants or offsprings thereof, wherein said offsprings include plant seeds or plant tissues.

39. Vectors comprising said gene of any one of claims 1-14 that encodes protein able to enhance heat resistance of plants or microorganisms.

40. Said vectors according to claim 39, **characterized in that** said vectors are expression vectors.

41. Said vectors according to claim 40, **characterized in that** said expression vectors are eukaryotic ones.

42. Host cells comprising said gene of any one of claims 1-14 that encodes protein able to enhance heat resistance of plants or microorganisms.

43. Said host cells according to claim 40, **characterized in that** said host cells are plant cells or microorganisms.


SEQUENCE LISTING

<110>  SICHUAN BIODESIGN GENE ENGINEERING CO., LTD.

<120>  GENES ENCODING PROTEINS ABLE TO ENHANCE HEAT RESISTANCE OF PLANTS AND
MICROORGANISMS AND USES THEREOF

<130>  P15474.EP-PCT

<150>  CN201010300890.x
<151>  2010-01-28

<160>  71

<170>  PatentIn version 3.4

<210>  1
<211>  867
<212>  DNA
<213>  artificial

<220>
<223>  sequence of Thermal Resistance Gene in Arabidopsis thaliana (AtTR1)

<400>  1
atggctgatc atttgagttt atgtaccgat cgtctcataa catctgagag cttggactca      60

gagaaagatt cagattcttc aggagaaagt tcttaccggc ctcaaggcac agacttggct     120

tcttcatctg tgaatgaaac tgaggttcct agagagtact acgcggttgc ggatgaggaa     180

gaacctcttc tccaatctgt tgagtgtcgt atttgccaag aggaagatag tactaagaac     240

cttgaagctc cttgtgcttg taatggtagt ttaaagtatg ctcaccgcaa gtgtgttcag     300

cgttggtgta atgagaaagg cgacataacc tgtgaaatat gtcaccagcc ttatcaacat     360

ggatacactg cacctccgcc tcctcctcct gatgaaacta taattcacat cggtgacgat     420

tgggagaacg gagttccgtt ggacttgacc gacccgcgca ttctggcaat ggcggctgca     480

gaacgtcatt tcttggaagc agactatgat gaatattctg agaataactc aagcggagct     540

gccttttgtc gctctgctgc tctcatccta atggcacttt tactgttacg agatgcgcta     600

aatctcacaa cgaacccaga cgacgaggac gatccaactg ccttcttctc tctattcctt     660

cttcgtgctg ctggttttct cctcccatgt tacatcatgg catgggccat cggtatacta     720

caacgtcgaa gacaaagaca ggaagcagcg gctctagctg ccgcagaagt tactttcatg     780

atacacggtg gtgggcctca acgcagagga ctgcactttg ctgtagcacc gcagccgccg     840

atatccaatg tcccaacacc agtttga                                         867

<210>  2
<211>  288
<212>  PRT
<213>  artificial

<220>
<223>  the amino acid sequence encoded by AtTR1

<400>  2

Met Ala Asp His Leu Ser Leu Cys Thr Asp Arg Leu Ile Thr Ser Glu
1               5                   10                  15

Ser Leu Asp Ser Glu Lys Asp Ser Asp Ser Ser Gly Glu Ser Ser Tyr
            20                  25                  30

Arg Pro Gln Gly Thr Asp Leu Ala Ser Ser Ser Val Asn Glu Thr Glu
        35                  40                  45

Val Pro Arg Glu Tyr Tyr Ala Val Ala Asp Glu Glu Glu Pro Leu Leu
    50                  55                  60

Gln Ser Val Glu Cys Arg Ile Cys Gln Glu Glu Asp Ser Thr Lys Asn
65                  70                  75                  80

Leu Glu Ala Pro Cys Ala Cys Asn Gly Ser Leu Lys Tyr Ala His Arg
                85                  90                  95

Lys Cys Val Gln Arg Trp Cys Asn Glu Lys Gly Asp Ile Thr Cys Glu
            100                 105                 110

Ile Cys His Gln Pro Tyr Gln His Gly Tyr Thr Ala Pro Pro Pro Pro
        115                 120                 125

Pro Pro Asp Glu Thr Ile Ile His Ile Gly Asp Asp Trp Glu Asn Gly
    130                 135                 140

Val Pro Leu Asp Leu Thr Asp Pro Arg Ile Leu Ala Met Ala Ala Ala
145                 150                 155                 160

Glu Arg His Phe Leu Glu Ala Asp Tyr Asp Glu Tyr Ser Glu Asn Asn
                165                 170                 175

Ser Ser Gly Ala Ala Phe Cys Arg Ser Ala Ala Leu Ile Leu Met Ala
            180                 185                 190

Leu Leu Leu Leu Arg Asp Ala Leu Asn Leu Thr Thr Asn Pro Asp Asp
        195                 200                 205

Glu Asp Asp Pro Thr Ala Phe Phe Ser Leu Phe Leu Leu Arg Ala Ala
    210                 215                 220

Gly Phe Leu Leu Pro Cys Tyr Ile Met Ala Trp Ala Ile Gly Ile Leu
225                 230                 235                 240

Gln Arg Arg Arg Gln Arg Gln Glu Ala Ala Ala Leu Ala Ala Ala Glu
                245                 250                 255

Val Thr Phe Met Ile His Gly Gly Gly Pro Gln Arg Arg Gly Leu His
            260                 265                 270

Phe Ala Val Ala Pro Gln Pro Pro Ile Ser Asn Val Pro Thr Pro Val
        275                 280                 285

<210>  3
<211>  24
<212>  DNA
<213>  artificial

<220>
<223>  artificial

<400>  3
atggctgatc atttgagttt atgt                                              24

<210>  4
<211>  26
<212>  DNA
<213>  artificial

<220>
<223>  artificial

<400>  4
tcaaactggt gttgggacat tggata                                            26

<210>  5
<211>  33
<212>  DNA
<213>  artificial

<220>
<223>  artificial

<400>  5
cgcggatcca tggctgatca tttgagttta tgt                                    33

<210>  6
<211>  35
<212>  DNA
<213>  artificial

<220>
<223>  artificial

<400>  6
ccggagctct caaactggtg ttgggacatt ggata                                  35

<210>  7
<211>  30
<212>  DNA
<213>  artificial

<220>
<223>  artificial

<400>  7

ttctccaatc tgttgagagt cgtatttgcc 30

<210> 8
<211> 30
<212> DNA
<213> artificial

<220>
<223> artificial

<400> 8
ggcaaatacg actctcaaca gattggagaa 30

<210> 9
<211> 32
<212> DNA
<213> artificial

<220>
<223> artificial

<400> 9
agtgtcgtat tggccaagag gaagatagta ct 32

<210> 10
<211> 32
<212> DNA
<213> artificial

<220>
<223> artificial

<400> 10
agtactatct tcctcttggc caatacgaca ct 32

<210> 11
<211> 23
<212> DNA
<213> artificial

<220>
<223> artificial

<400> 11
cttgaagctc ctagtgcttg taa 23

<210> 12
<211> 23
<212> DNA
<213> artificial

<220>
<223> artificial

<400> 12
ttacaagcac taggagcttc aag 23

<210> 13
<211> 32

<212>  DNA
<213>  artificial

<220>
<223>  artificial

<400>  13
gaagctcctt gtgctcgtaa tggtagttta aa                                    32


<210>  14
<211>  32
<212>  DNA
<213>  artificial

<220>
<223>  artificial

<400>  14
tttaaactac cattacgagc acaaggagct tc                                    32


<210>  15
<211>  27
<212>  DNA
<213>  artificial

<220>
<223> artificial

<400>  15
taaagtatgc taaccgcaag tgtgttc                                          27


<210>  16
<211>  27
<212>  DNA
<213>  artificial

<220>
<223>  artificial

<400>  16
gaacacactt gcggttagca tacttta                                          27


<210>  17
<211>  27
<212>  DNA
<213>  artificial

<220>
<223>  artificial

<400>  17
accgcaagta tgttcagcgt tggtgta                                          27


<210>  18
<211>  27
<212>  DNA
<213>  artificial

<220>
<223>  artificial

<400>  18
tacaccaacg ctgaacatac ttgcggt                                          27


<210>  19
<211>  867
<212>  DNA
<213>  artificial

<220>
<223>  SEQ ID NO: 2 N-terminal Cys69 substituted by Ser


<400>  19
atggctgatc atttgagttt atgtaccgat cgtctcataa catctgagag cttggactca      60

gagaaagatt cagattcttc aggagaaagt tcttaccggc ctcaaggcac agacttggct     120

tcttcatctg tgaatgaaac tgaggttcct agagagtact acgcggttgc ggatgaggaa     180

gaacctcttc tccaatctgt tgagagtcgt atttgccaag aggaagatag tactaagaac     240

cttgaagctc cttgtgcttg taatggtagt ttaaagtatg ctcaccgcaa gtgtgttcag     300

cgttggtgta atgagaaagg cgacataacc tgtgaaatat gtcaccagcc ttatcaacat     360

ggatacactg cacctccgcc tcctcctcct gatgaaacta taattcacat cggtgacgat     420

tgggagaacg gagttccgtt ggacttgacc gacccgcgca ttctggcaat ggcggctgca     480

gaacgtcatt tcttggaagc agactatgat gaatattctg agaataactc aagcggagct     540

gccttttgtc gctctgctgc tctcatccta atggcacttt tactgttacg agatgcgcta     600

aatctcacaa cgaacccaga cgacgaggac gatccaactg ccttcttctc tctattcctt     660

cttcgtgctg ctggttttct cctcccatgt tacatcatgg catgggccat cggtatacta     720

caacgtcgaa gacaaagaca ggaagcagcg gctctagctg ccgcagaagt tactttcatg     780

atacacggtg gtgggcctca acgcagagga ctgcactttg ctgtagcacc gcagccgccg     840

atatccaatg tcccaacacc agtttga                                         867


<210>  20
<211>  867
<212>  DNA
<213>  artificial

<220>
<223>  SEQ ID NO: 2 N-terminal Cys72 substituted by Gly

<400>  20
atggctgatc atttgagttt atgtaccgat cgtctcataa catctgagag cttggactca      60

gagaaagatt cagattcttc aggagaaagt tcttaccggc ctcaaggcac agacttggct     120

tcttcatctg tgaatgaaac tgaggttcct agagagtact acgcggttgc ggatgaggaa     180

gaacctcttc tccaatctgt tgagtgtcgt attggccaag aggaagatag tactaagaac     240

cttgaagctc cttgtgcttg taatggtagt ttaaagtatg ctcaccgcaa gtgtgttcag     300

cgttggtgta atgagaaagg cgacataacc tgtgaaatat gtcaccagcc ttatcaacat 360

ggatacactg cacctccgcc tcctcctcct gatgaaacta taattcacat cggtgacgat 420

tgggagaacg gagttccgtt ggacttgacc gacccgcgca ttctggcaat ggcggctgca 480

gaacgtcatt tcttggaagc agactatgat gaatattctg agaataactc aagcggagct 540

gccttttgtc gctctgctgc tctcatccta atggcacttt tactgttacg agatgcgcta 600

aatctcacaa cgaacccaga cgacgaggac gatccaactg ccttcttctc tctattcctt 660

cttcgtgctg ctggttttct cctcccatgt tacatcatgg catgggccat cggtatacta 720

caacgtcgaa gacaaagaca ggaagcagcg gctctagctg ccgcagaagt tactttcatg 780

atacacggtg gtgggcctca acgcagagga ctgcactttg ctgtagcacc gcagccgccg 840

atatccaatg tcccaacacc agtttga 867

<210> 21
<211> 867
<212> DNA
<213> artificial

<220>
<223> SEQ ID NO: 2 N-terminal Cys85 substituted by Ser

<400> 21
atggctgatc atttgagttt atgtaccgat cgtctcataa catctgagag cttggactca 60

gagaaagatt cagattcttc aggagaaagt tcttaccggc ctcaaggcac agacttggct 120

tcttcatctg tgaatgaaac tgaggttcct agagagtact acgcggttgc ggatgaggaa 180

gaacctcttc tccaatctgt tgagtgtcgt atttgccaag aggaagatag tactaagaac 240

cttgaagctc ctagtgcttg taatggtagt ttaaagtatg ctcaccgcaa gtgtgttcag 300

cgttggtgta atgagaaagg cgacataacc tgtgaaatat gtcaccagcc ttatcaacat 360

ggatacactg cacctccgcc tcctcctcct gatgaaacta taattcacat cggtgacgat 420

tgggagaacg gagttccgtt ggacttgacc gacccgcgca ttctggcaat ggcggctgca 480

gaacgtcatt tcttggaagc agactatgat gaatattctg agaataactc aagcggagct 540

gccttttgtc gctctgctgc tctcatccta atggcacttt tactgttacg agatgcgcta 600

aatctcacaa cgaacccaga cgacgaggac gatccaactg ccttcttctc tctattcctt 660

cttcgtgctg ctggttttct cctcccatgt tacatcatgg catgggccat cggtatacta 720

caacgtcgaa gacaaagaca ggaagcagcg gctctagctg ccgcagaagt tactttcatg 780

atacacggtg gtgggcctca acgcagagga ctgcactttg ctgtagcacc gcagccgccg 840

atatccaatg tcccaacacc agtttga 867

<210> 22
<211> 867
<212> DNA
<213> artificial

<220>
<223>  SEQ ID NO: 2 N-terminal Cys87 substituted by Arg

<400>  22
atggctgatc atttgagttt atgtaccgat cgtctcataa catctgagag cttggactca      60

gagaaagatt cagattcttc aggagaaagt tcttaccggc ctcaaggcac agacttggct     120

tcttcatctg tgaatgaaac tgaggttcct agagagtact acgcggttgc ggatgaggaa     180

gaacctcttc tccaatctgt tgagtgtcgt atttgccaag aggaagatag tactaagaac     240

cttgaagctc cttgtgctcg taatggtagt ttaaagtatg ctcaccgcaa gtgtgttcag     300

cgttggtgta atgagaaagg cgacataacc tgtgaaatat gtcaccagcc ttatcaacat     360

ggatacactg cacctccgcc tcctcctcct gatgaaacta taattcacat cggtgacgat     420

tgggagaacg gagttccgtt ggacttgacc gacccgcgca ttctggcaat ggcggctgca     480

gaacgtcatt tcttggaagc agactatgat gaatattctg agaataactc aagcggagct     540

gccttttgtc gctctgctgc tctcatccta atggcacttt tactgttacg agatgcgcta     600

aatctcacaa cgaacccaga cgacgaggac gatccaactg ccttcttctc tctattcctt     660

cttcgtgctg ctggttttct cctcccatgt tacatcatgg catgggccat cggtatacta     720

caacgtcgaa gacaaagaca ggaagcagcg gctctagctg ccgcagaagt tactttcatg     780

atacacggtg gtgggcctca acgcagagga ctgcactttg ctgtagcacc gcagccgccg     840

atatccaatg tcccaacacc agtttga                                         867


<210>  23
<211>  867
<212>  DNA
<213>  artificial

<220>
<223>  SEQ ID NO: 2 N-terminal His95 substituted by Asn


<400>  23
atggctgatc atttgagttt atgtaccgat cgtctcataa catctgagag cttggactca      60

gagaaagatt cagattcttc aggagaaagt tcttaccggc ctcaaggcac agacttggct     120

tcttcatctg tgaatgaaac tgaggttcct agagagtact acgcggttgc ggatgaggaa     180

gaacctcttc tccaatctgt tgagtgtcgt atttgccaag aggaagatag tactaagaac     240

cttgaagctc cttgtgcttg taatggtagt ttaaagtatg ctaaccgcaa gtgtgttcag     300

cgttggtgta atgagaaagg cgacataacc tgtgaaatat gtcaccagcc ttatcaacat     360

ggatacactg cacctccgcc tcctcctcct gatgaaacta taattcacat cggtgacgat     420

tgggagaacg gagttccgtt ggacttgacc gacccgcgca ttctggcaat ggcggctgca     480

gaacgtcatt tcttggaagc agactatgat gaatattctg agaataactc aagcggagct     540

gccttttgtc gctctgctgc tctcatccta atggcacttt tactgttacg agatgcgcta     600

aatctcacaa cgaacccaga cgacgaggac gatccaactg ccttcttctc tctattcctt 660

cttcgtgctg ctggttttct cctcccatgt tacatcatgg catgggccat cggtatacta 720

caacgtcgaa gacaaagaca ggaagcagcg gctctagctg ccgcagaagt tactttcatg 780

atacacggtg gtgggcctca acgcagagga ctgcactttg ctgtagcacc gcagccgccg 840

atatccaatg tcccaacacc agtttga 867

<210> 24
<211> 867
<212> DNA
<213> artificial

<220>
<223> SEQ ID NO: 2 N-terminal 98Cys substituted by Tyr

<400> 24
atggctgatc atttgagttt atgtaccgat cgtctcataa catctgagag cttggactca 60

gagaaagatt cagattcttc aggagaaagt tcttaccggc ctcaaggcac agacttggct 120

tcttcatctg tgaatgaaac tgaggttcct agagagtact acgcggttgc ggatgaggaa 180

gaacctcttc tccaatctgt tgagtgtcgt atttgccaag aggaagatag tactaagaac 240

cttgaagctc cttgtgcttg taatggtagt ttaaagtatg ctcaccgcaa gtatgttcag 300

cgttggtgta atgagaaagg cgacataacc tgtgaaatat gtcaccagcc ttatcaacat 360

ggatacactg cacctccgcc tcctcctcct gatgaaacta taattcacat cggtgacgat 420

tgggagaacg gagttccgtt ggacttgacc gacccgcgca ttctggcaat ggcggctgca 480

gaacgtcatt tcttggaagc agactatgat gaatattctg agaataactc aagcggagct 540

gccttttgtc gctctgctgc tctcatccta atggcacttt tactgttacg agatgcgcta 600

aatctcacaa cgaacccaga cgacgaggac gatccaactg ccttcttctc tctattcctt 660

cttcgtgctg ctggttttct cctcccatgt tacatcatgg catgggccat cggtatacta 720

caacgtcgaa gacaaagaca ggaagcagcg gctctagctg ccgcagaagt tactttcatg 780

atacacggtg gtgggcctca acgcagagga ctgcactttg ctgtagcacc gcagccgccg 840

atatccaatg tcccaacacc agtttga 867

<210> 25
<211> 24
<212> DNA
<213> artificial

<220>
<223> artificial

<400> 25
tgatcatttg agtttatgta ccga 24

<210>  26
<211>  25
<212>  DNA
<213>  artificial

<220>
<223>  artificial

<400>  26
tcaaactggt gttgggacat tggat                                          25


<210>  27
<211>  26
<212>  DNA
<213>  artificial

<220>
<223>  artificial

<400>  27
aatctgttga gagtcgtatt tgccaa                                         26


<210>  28
<211>  25
<212>  DNA
<213>  artificial

<220>
<223>  artificial

<400>  28
tcaaactggt gttgggacat tggat                                          25


<210>  29
<211>  25
<212>  DNA
<213>  artificial

<220>
<223>  artificial

<400>  29
gtcgtattgg ccaagaggaa gatag                                          25


<210>  30
<211>  25
<212>  DNA
<213>  artificial

<220>
<223>  artificial

<400>  30
tcaaactggt gttgggacat tggat                                          25


<210>  31
<211>  24
<212>  DNA
<213>  artificial

<220>
<223>  artificial

<400>  31
cttgaagctc ctagtgcttg taat                                              24


<210>  32
<211>  25
<212>  DNA
<213>  artificial

<220>
<223>  artificial

<400>  32
tcaaactggt gttgggacat tggat                                             25


<210>  33
<211>  27
<212>  DNA
<213>  artificial

<220>
<223>  artificial

<400>  33
cttgtgctcg taatggtagt ttaaagt                                           27


<210>  34
<211>  25
<212>  DNA
<213>  artificial

<220>
<223>  artificial

<400>  34
tcaaactggt gttgggacat tggat                                             25


<210>  35
<211>  26
<212>  DNA
<213>  artificial

<220>
<223>  artificial

<400>  35
aagtatgcta accgcaagtg tgttct                                            26


<210>  36
<211>  25
<212>  DNA
<213>  artificial

<220>
<223>  artificial

<400>  36
tcaaactggt gttgggacat tggat                                             25

<210>  37
<211>  29
<212>  DNA
<213>  artificial

<220>
<223>  artificial

<400>  37
tatgctcacc gcaagtatgt tcagcgttt                                          29


<210>  38
<211>  24
<212>  DNA
<213>  artificial

<220>
<223>  artificial

<400>  38
tcagactggt gttgggttgg atat                                               24


<210>  39
<211>  996
<212>  DNA
<213>  artificial

<220>
<223>  a homologous gene of AtTR1 from Ricinus communis


<220>
<221>  CDS
<222>  (68)..(946)
<223>  the amino acid sequence encoded by RcTR1

<400>  39
ttgacgtttg gactctgata agaaaattat tatgatgtgg tgtagcagga tttttaatca      60

aatacca atg agt gat caa cta gtt ttg tgt gct gat cgt ctc ata aca       109
        Met Ser Asp Gln Leu Val Leu Cys Ala Asp Arg Leu Ile Thr
        1               5                   10

cct gaa agt ttg caa tca atg gaa aaa gct aag gag cca gga tct tct       157
Pro Glu Ser Leu Gln Ser Met Glu Lys Ala Lys Glu Pro Gly Ser Ser
15                  20                  25                  30

ggg gag tgt tca tct tcc cat act gct gat cta ccc act tgt gta att       205
Gly Glu Cys Ser Ser Ser His Thr Ala Asp Leu Pro Thr Cys Val Ile
                35                  40                  45

gat gtt gag gga ggg ggg gaa cat ggt gta tct gaa gaa gaa gaa cca       253
Asp Val Glu Gly Gly Gly Glu His Gly Val Ser Glu Glu Glu Glu Pro
            50                  55                  60

ctt ctg caa aca atg gaa tgt cgc att tgc cag gag gag gat agc att       301
Leu Leu Gln Thr Met Glu Cys Arg Ile Cys Gln Glu Glu Asp Ser Ile
        65                  70                  75

aat aat ttg gag gca cca tgt gcc tgt agt ggt agt ttg aag ttt gcc       349
Asn Asn Leu Glu Ala Pro Cys Ala Cys Ser Gly Ser Leu Lys Phe Ala

80                  85                  90

cac agg aaa tgt gtt cag cga tgg tgc aac gaa aaa gga gat ata acc          397
His Arg Lys Cys Val Gln Arg Trp Cys Asn Glu Lys Gly Asp Ile Thr
95                  100                 105                 110

tgt gag ata tgt cat cag cct tac caa cct aat tac act gcc tca cca          445
Cys Glu Ile Cys His Gln Pro Tyr Gln Pro Asn Tyr Thr Ala Ser Pro
                115                 120                 125

cct ctt cca tta gaa gat aca gca att gat atc agt gag ggt tgg act          493
Pro Leu Pro Leu Glu Asp Thr Ala Ile Asp Ile Ser Glu Gly Trp Thr
            130                 135                 140

att gct ggg act cct ttg gat ttg cac gat cct cga att ttg gct atg          541
Ile Ala Gly Thr Pro Leu Asp Leu His Asp Pro Arg Ile Leu Ala Met
        145                 150                 155

gct gca gca gag cgt cat ttt ttg gag gct gaa tat gat gaa tat gct          589
Ala Ala Ala Glu Arg His Phe Leu Glu Ala Glu Tyr Asp Glu Tyr Ala
    160                 165                 170

gat tcc agt gct agt gga gct gca ttt tgc cgt tct gct gct tta att          637
Asp Ser Ser Ala Ser Gly Ala Ala Phe Cys Arg Ser Ala Ala Leu Ile
175                 180                 185                 190

cta atg gct ctt ctg ctc ctg agg cat gct atg tct ctt act gga gat          685
Leu Met Ala Leu Leu Leu Leu Arg His Ala Met Ser Leu Thr Gly Asp
                195                 200                 205

agt gat gaa gat gct tct acg ttt ttc tct ctt ttc ctg ata cgg gct          733
Ser Asp Glu Asp Ala Ser Thr Phe Phe Ser Leu Phe Leu Ile Arg Ala
            210                 215                 220

gct gga ttt ctt ctc cca tgt tac atc atg gcc tgg gcc atc agc ata          781
Ala Gly Phe Leu Leu Pro Cys Tyr Ile Met Ala Trp Ala Ile Ser Ile
        225                 230                 235

ttg cag aga cga agg caa aga cag gaa gca gca gca ctt gca gct act          829
Leu Gln Arg Arg Arg Gln Arg Gln Glu Ala Ala Ala Leu Ala Ala Thr
    240                 245                 250

gag gtt gca ttc atg cta cag gca ggt caa cga cgg gga ttg caa ttc          877
Glu Val Ala Phe Met Leu Gln Ala Gly Gln Arg Arg Gly Leu Gln Phe
255                 260                 265                 270

aca att gca cct gga ctt gca gtg aat cct cac caa gct gca act cca          925
Thr Ile Ala Pro Gly Leu Ala Val Asn Pro His Gln Ala Ala Thr Pro
                275                 280                 285

cag caa gag cca ctt caa tga tgaaatgaat ggggatgcta gtacagaagc          976
Gln Gln Glu Pro Leu Gln
            290

atgagtgatc atcttacttg                                                    996

<210>  40
<211>  292
<212>  PRT
<213>  artificial

<220>
<223>  Synthetic Construct

<400> 40
   Met Ser Asp Gln Leu Val Leu Cys Ala Asp Arg Leu Ile Thr Pro Glu
   1               5                   10                  15
   Ser Leu Gln Ser Met Glu Lys Ala Lys Glu Pro Gly Ser Ser Gly Glu
               20                  25                  30
   Cys Ser Ser Ser His Thr Ala Asp Leu Pro Thr Cys Val Ile Asp Val
           35                  40                  45
   Glu Gly Gly Gly Glu His Gly Val Ser Glu Glu Glu Glu Pro Leu Leu
       50                  55                  60
   Gln Thr Met Glu Cys Arg Ile Cys Gln Glu Glu Asp Ser Ile Asn Asn
   65                  70                  75                  80
   Leu Glu Ala Pro Cys Ala Cys Ser Gly Ser Leu Lys Phe Ala His Arg
                   85                  90                  95
   Lys Cys Val Gln Arg Trp Cys Asn Glu Lys Gly Asp Ile Thr Cys Glu
               100                 105                 110
   Ile Cys His Gln Pro Tyr Gln Pro Asn Tyr Thr Ala Ser Pro Pro Leu
           115                 120                 125
   Pro Leu Glu Asp Thr Ala Ile Asp Ile Ser Glu Gly Trp Thr Ile Ala
       130                 135                 140
   Gly Thr Pro Leu Asp Leu His Asp Pro Arg Ile Leu Ala Met Ala Ala
   145                 150                 155                 160
   Ala Glu Arg His Phe Leu Glu Ala Glu Tyr Asp Glu Tyr Ala Asp Ser
                   165                 170                 175
   Ser Ala Ser Gly Ala Ala Phe Cys Arg Ser Ala Ala Leu Ile Leu Met
               180                 185                 190
   Ala Leu Leu Leu Leu Arg His Ala Met Ser Leu Thr Gly Asp Ser Asp
           195                 200                 205
   Glu Asp Ala Ser Thr Phe Phe Ser Leu Phe Leu Ile Arg Ala Ala Gly
       210                 215                 220
   Phe Leu Leu Pro Cys Tyr Ile Met Ala Trp Ala Ile Ser Ile Leu Gln
   225                 230                 235                 240
   Arg Arg Arg Gln Arg Gln Glu Ala Ala Ala Leu Ala Ala Thr Glu Val
                   245                 250                 255
   Ala Phe Met Leu Gln Ala Gly Gln Arg Arg Gly Leu Gln Phe Thr Ile
   260 265 270
   Ala Pro Gly Leu Ala Val Asn Pro His Gln Ala Ala Thr Pro Gln Gln
   275 280 285
   Glu Pro Leu Gln
   290

<210>  41
<211>  23
<212>  DNA
<213>  artificial

<220>
<223>  artificial

<400>  41
gtggtgtagc aggattttta atc                                              23

<210>  42
<211>  19
<212>  DNA
<213>  artificial

<220>
<223>  artificial

<400>  42
gcatccccat tcatttcat                                                   19

<210>  43
<211>  30
<212>  DNA
<213>  artificial

<220>
<223>  artificial

<400>  43
ccggaattca tgagtgatca actagttttg                                       30

<210>  44
<211>  27
<212>  DNA
<213>  artificial

<220>
<223>  artificial

<400>  44
cccaagcttt cattgaagtg gctcttg                                          27

<210>  45
<211>  1301
<212>  DNA
<213>  artificial

<220>
<223>  a homologous gene of AtTR1 from Oryza sativa(OsTR1-1)


<220>
<221>  CDS
<222>  (96)..(848)
<223>  the amino acid sequence encoded by OsTR1-1

<400>  45
gaggaggagt gcttcccagc tcattccgcc gaggtcgtga gagctgctga gcgtggagat      60

tttttcttta aaactttttt gggtgatttg ctgga atg ggg gat cat gtt gcg      113
                                       Met Gly Asp His Val Ala
                                       1               5

gtg gat gtt ggg ggg ctc atg gcg tcc cgg ggc gcc ggc gag gag gaa      161
Val Asp Val Gly Gly Leu Met Ala Ser Arg Gly Ala Gly Glu Glu Glu
            10                  15                  20

acg ggg gcg ctg att ggg atg gtg gaa tgc cgc att tgc cag gag gag      209
Thr Gly Ala Leu Ile Gly Met Val Glu Cys Arg Ile Cys Gln Glu Glu
        25                  30                  35

gac ctc gcc aag aac ctc gag agc ccc tgc gct tgt agc ggt agc ctc      257
Asp Leu Ala Lys Asn Leu Glu Ser Pro Cys Ala Cys Ser Gly Ser Leu
    40                  45                  50

aag tac gct cat aga gaa tgt gtg caa aga tgg tgc aat gag aaa gga      305
Lys Tyr Ala His Arg Glu Cys Val Gln Arg Trp Cys Asn Glu Lys Gly
55                  60                  65                  70

gac ata atc tgt gaa atc tgc cac gtg tca tac aag cct ggc tat act      353
Asp Ile Ile Cys Glu Ile Cys His Val Ser Tyr Lys Pro Gly Tyr Thr
                75                  80                  85

gcc cca ccc cag gta cat cat gat gag act act ata gag atc agt agt      401
Ala Pro Pro Gln Val His His Asp Glu Thr Thr Ile Glu Ile Ser Ser
            90                  95                  100

gga gat tgg tct atc tct ggc aat cgg ttg gat tta cat gat cct aga      449
Gly Asp Trp Ser Ile Ser Gly Asn Arg Leu Asp Leu His Asp Pro Arg
        105                 110                 115

atc ttg gct atg gct gcc gct caa cac cgt tta ctt gaa gat gag tat      497
Ile Leu Ala Met Ala Ala Ala Gln His Arg Leu Leu Glu Asp Glu Tyr
    120                 125                 130

gat gaa tat act gca aca aac aac aat gct gct gcc ttt tgc cgc tcc      545
Asp Glu Tyr Thr Ala Thr Asn Asn Asn Ala Ala Ala Phe Cys Arg Ser
135                 140                 145                 150

ata ttt cta att cta atg gct ctt cta ctc tta agg cat aca cta acc      593
Ile Phe Leu Ile Leu Met Ala Leu Leu Leu Leu Arg His Thr Leu Thr
                155                 160                 165

att act agt agt gat gat gaa gat gac gca tct gct att ttc tcg ttg      641
Ile Thr Ser Ser Asp Asp Glu Asp Asp Ala Ser Ala Ile Phe Ser Leu
            170                 175                 180

ttt ctt ttg agg gct gct gga ttt ttg ctg cca tgc tat atc atg gct      689
Phe Leu Leu Arg Ala Ala Gly Phe Leu Leu Pro Cys Tyr Ile Met Ala
        185                 190                 195

tgg gct att agt atc atg caa cgt caa aga caa aga cag gaa gaa gca     737
Trp Ala Ile Ser Ile Met Gln Arg Gln Arg Gln Arg Gln Glu Glu Ala
    200                 205                 210

atg ctt cta cca act gaa gtg gca atc ata ctc cac cga aat gga agg     785
Met Leu Leu Pro Thr Glu Val Ala Ile Ile Leu His Arg Asn Gly Arg
215                 220                 225                 230

act atg caa ttc gcc gta gcg cca gaa tct cct acc aca cct cat cca     833
Thr Met Gln Phe Ala Val Ala Pro Glu Ser Pro Thr Thr Pro His Pro
                235                 240                 245

gaa caa ggg caa tag aatttgctgg caacaggcat atcttgtgag tgcatacaag     888
Glu Gln Gly Gln
            250

ataccttgag ccttctacac cctgactctg tgtacactgc aagccggttg caccagccac     948

tttcaatggc caaattgcag caaaaaagtg ttgcgtgtct atctctcgat ttgcataaac    1008

tttgcagcta ggcttgaatg cttttgtgat tggctttcag cttcttctgc acataaagag    1068

attcctttca ataaatgaat ataagctaaa ttaacaagaa gaattcttgt tccagggtca    1128

ctagacttgt tcatggcttc gtgctgcgac gggccagcta ctgttccaac aaatccggag    1188

cttttttttt tttcgtcttg gtctgtttac caattccttc tgttttgaga accacaattt    1248

tcacgtctgt ggaagcctgt aagctctaca cacgcaagcc atgcttcaat tgc           1301

<210>  46
<211>  250
<212>  PRT
<213>  artificial

<220>
<223>  Synthetic Construct

<400>  46

Met Gly Asp His Val Ala Val Asp Val Gly Gly Leu Met Ala Ser Arg
1               5                   10                  15

Gly Ala Gly Glu Glu Glu Thr Gly Ala Leu Ile Gly Met Val Glu Cys
            20                  25                  30

Arg Ile Cys Gln Glu Glu Asp Leu Ala Lys Asn Leu Glu Ser Pro Cys
        35                  40                  45

Ala Cys Ser Gly Ser Leu Lys Tyr Ala His Arg Glu Cys Val Gln Arg
    50                  55                  60

Trp Cys Asn Glu Lys Gly Asp Ile Ile Cys Glu Ile Cys His Val Ser
65                  70                  75                  80

Tyr Lys Pro Gly Tyr Thr Ala Pro Pro Gln Val His His Asp Glu Thr
                85                  90                  95

Thr Ile Glu Ile Ser Ser Gly Asp Trp Ser Ile Ser Gly Asn Arg Leu
            100                 105                 110

Asp Leu His Asp Pro Arg Ile Leu Ala Met Ala Ala Ala Gln His Arg
        115                 120                 125

Leu Leu Glu Asp Glu Tyr Asp Glu Tyr Thr Ala Thr Asn Asn Asn Ala
    130                 135                 140

Ala Ala Phe Cys Arg Ser Ile Phe Leu Ile Leu Met Ala Leu Leu Leu
145                 150                 155                 160

Leu Arg His Thr Leu Thr Ile Thr Ser Ser Asp Asp Glu Asp Asp Ala
                165                 170                 175

Ser Ala Ile Phe Ser Leu Phe Leu Leu Arg Ala Ala Gly Phe Leu Leu
            180                 185                 190

Pro Cys Tyr Ile Met Ala Trp Ala Ile Ser Ile Met Gln Arg Gln Arg
        195                 200                 205

Gln Arg Gln Glu Glu Ala Met Leu Leu Pro Thr Glu Val Ala Ile Ile
    210                 215                 220

Leu His Arg Asn Gly Arg Thr Met Gln Phe Ala Val Ala Pro Glu Ser
225                 230                 235                 240

Pro Thr Thr Pro His Pro Glu Gln Gly Gln
                245                 250

<210>  47
<211>  1504
<212>  DNA
<213>  artificial

<220>
<223>  a homologous gene of AtTR1 from Oryza sativa(OsTR1-2)

<220>
<221>  CDS
<222>  (291)..(1127)
<223>  the amino acid sequence encoded by OsTR1-2

<400>  47
gaacgaaaca cgccttcttc cccaacgacc aaaccaaagt ttgggtgaag aaaaaaaaat      60

atcgccgcgt ttttatcccc cacgagacga gtcgagacga ccaaaagcct aactaatcgg      120

gggcaagacg aggaggagag cggattctcg tcggcttcgg cgaccggaag aggagaggag      180

accgaccccg cgccgtgttc cgccgccgcc gccgccgccg acgacgacga cggcaagtag      240

gaatttgatt ctcctgggga attaacttgt taggagaaaa ggaatttggg atg ggc      296
                                                       Met Gly

                                                       1

gac cat gtg gtg gtg aat gtg gaa ggc ttg gga aac gat gga ggg gct 344
Asp His Val Val Val Asn Val Glu Gly Leu Gly Asn Asp Gly Gly Ala
        5                   10                  15

gtg gag aag cca tct gag gcc gtg aac agc tca gtg gtg gct gct gcg 392
Val Glu Lys Pro Ser Glu Ala Val Asn Ser Ser Val Val Ala Ala Ala
    20                  25                  30

tcg ctt tct acg act gtt gat acg gtc gag gag ggc ggt gga gag gag 440
Ser Leu Ser Thr Thr Val Asp Thr Val Glu Glu Gly Gly Gly Glu Glu
35                  40                  45                  50

gag ccg ctc atc cag gcg gcg gag tgc cgt ata tgc cag gag gag gat 488
Glu Pro Leu Ile Gln Ala Ala Glu Cys Arg Ile Cys Gln Glu Glu Asp
                55                  60                  65

agc atc aag aac ctc gag aag cct tgt gct tgc agt ggc agc ctc aag 536
Ser Ile Lys Asn Leu Glu Lys Pro Cys Ala Cys Ser Gly Ser Leu Lys
            70                  75                  80

tat gct cat aga gct tgt gtg caa cgc tgg tgc aat gag aaa gga gac 584
Tyr Ala His Arg Ala Cys Val Gln Arg Trp Cys Asn Glu Lys Gly Asp
        85                  90                  95

att aca tgt gaa atc tgt cat gag caa tat aag cat gga tac aca gct 632
Ile Thr Cys Glu Ile Cys His Glu Gln Tyr Lys His Gly Tyr Thr Ala
    100                 105                 110

cca cct cgt gtt gaa cca gac gat acc atc ata gat atc ggt ggt gat 680
Pro Pro Arg Val Glu Pro Asp Asp Thr Ile Ile Asp Ile Gly Gly Asp
115                 120                 125                 130

tgg gca cgt gac cca aga att att gct gta gcg gct gca caa cgt cgt 728
Trp Ala Arg Asp Pro Arg Ile Ile Ala Val Ala Ala Ala Gln Arg Arg
                135                 140                 145

ctt ctt gaa act gag tat gat gaa tat gct ggt act gat gct agt ggt 776
Leu Leu Glu Thr Glu Tyr Asp Glu Tyr Ala Gly Thr Asp Ala Ser Gly
            150                 155                 160

gct gca ttc tgc cgt tct gct gca ctt ata cta atg gcc ttg ttg cta 824
Ala Ala Phe Cys Arg Ser Ala Ala Leu Ile Leu Met Ala Leu Leu Leu
        165                 170                 175

ttg agg cat gct ttg tct atc tca gac aat gaa gga gat gat gat gct 872
Leu Arg His Ala Leu Ser Ile Ser Asp Asn Glu Gly Asp Asp Asp Ala
    180                 185                 190

tct acc atg ttc tcg ctc ttt cta ctt cga gct gct gga ttt ctg ctg 920
Ser Thr Met Phe Ser Leu Phe Leu Leu Arg Ala Ala Gly Phe Leu Leu
195                 200                 205                 210

cca tgc tat atc atg gct tgg ata ttc agt att ttg cat cgc cgg cga 968
Pro Cys Tyr Ile Met Ala Trp Ile Phe Ser Ile Leu His Arg Arg Arg
                215                 220                 225

caa aga cag gaa gcg gct gca att gct gca gca gag gtg gca ttc atc 1016
Gln Arg Gln Glu Ala Ala Ala Ile Ala Ala Ala Glu Val Ala Phe Ile
            230                 235                 240

ctg caa tca gcc caa ggc cat gcc ctt cag ttc acc atc gct cca gac 1064
Leu Gln Ser Ala Gln Gly His Ala Leu Gln Phe Thr Ile Ala Pro Asp
        245                 250                 255

tcc ccc gcc act cca cag cat gag cca cag cag cag cag ccg cag ccg     1112
Ser Pro Ala Thr Pro Gln His Glu Pro Gln Gln Gln Gln Pro Gln Pro
    260                 265                 270

cag ccg cag caa tag ctagttactt cagtagcgtc tctcaactac tgactcgcac     1167
Gln Pro Gln Gln
275

atgcagaaga agaggatacc tcagctagtg gccgctttgt tgtttcctgt tgcaactggc     1227

ccagggacgc taagaacctg gcctcgcctt gtgtggccgg gtagaaacct tgtaaaacgc     1287

cagagattca tttgttgcaa gcgaccgcag cttaagcccc gctacccttc gatttgtctc     1347

tgtgtaactc aagcattgat ttattgtaaa tttgtcgtct gttggagtcc atttttttag     1407

ttcctttgcc aagtgtttta ctgccatgta cagtattatt acatgggagc ttttatcttt     1467

caaagaatta atctgagtga gtgtgaaact gttaggc                            1504

<210>  48
<211>  278
<212>  PRT
<213>  artificial

<220>
<223>  Synthetic Construct

<400>  48

Met Gly Asp His Val Val Val Asn Val Glu Gly Leu Gly Asn Asp Gly
1               5                   10                  15

Gly Ala Val Glu Lys Pro Ser Glu Ala Val Asn Ser Ser Val Val Ala
            20                  25                  30

Ala Ala Ser Leu Ser Thr Thr Val Asp Thr Val Glu Glu Gly Gly Gly
        35                  40                  45

Glu Glu Glu Pro Leu Ile Gln Ala Ala Glu Cys Arg Ile Cys Gln Glu
    50                  55                  60

Glu Asp Ser Ile Lys Asn Leu Glu Lys Pro Cys Ala Cys Ser Gly Ser
65                  70                  75                  80

Leu Lys Tyr Ala His Arg Ala Cys Val Gln Arg Trp Cys Asn Glu Lys
                85                  90                  95

Gly Asp Ile Thr Cys Glu Ile Cys His Glu Gln Tyr Lys His Gly Tyr
            100                 105                 110

Thr Ala Pro Pro Arg Val Glu Pro Asp Asp Thr Ile Ile Asp Ile Gly
        115                 120                 125

Gly Asp Trp Ala Arg Asp Pro Arg Ile Ile Ala Val Ala Ala Ala Gln

130                 135                 140

Arg Arg Leu Leu Glu Thr Glu Tyr Asp Glu Tyr Ala Gly Thr Asp Ala
145                 150                 155                 160

Ser Gly Ala Ala Phe Cys Arg Ser Ala Ala Leu Ile Leu Met Ala Leu
                165                 170                 175

Leu Leu Leu Arg His Ala Leu Ser Ile Ser Asp Asn Glu Gly Asp Asp
            180                 185                 190

Asp Ala Ser Thr Met Phe Ser Leu Phe Leu Leu Arg Ala Ala Gly Phe
        195                 200                 205

Leu Leu Pro Cys Tyr Ile Met Ala Trp Ile Phe Ser Ile Leu His Arg
    210                 215                 220

Arg Arg Gln Arg Gln Glu Ala Ala Ala Ile Ala Ala Ala Glu Val Ala
225                 230                 235                 240

Phe Ile Leu Gln Ser Ala Gln Gly His Ala Leu Gln Phe Thr Ile Ala
                245                 250                 255

Pro Asp Ser Pro Ala Thr Pro Gln His Glu Pro Gln Gln Gln Gln Pro
            260                 265                 270

Gln Pro Gln Pro Gln Gln
        275

<210>  49
<211>  21
<212>  DNA
<213>  artificial

<220>
<223>  artificial

<400>  49
aaactttttt gggtgatttg c                                                21

<210>  50
<211>  22
<212>  DNA
<213>  artificial

<220>
<223>  artificial

<400>  50
caaattctat tgcccttgtt ct                                               22

<210>  51
<211>  21

<212>  DNA
<213>  artificial

<220>
<223>  artificial

<400>  51
ggaatttggg atgggcgacc a                                                21


<210>  52
<211>  27
<212>  DNA
<213>  artificial

<220>
<223>  artificial

<400>  52
agagacgcta ctgaagtaac tagctat                                          27


<210>  53
<211>  33
<212>  DNA
<213>  artificial

<220>
<223>  artificial

<400>  53
cgcggatcca tgggggatca tgttgcggtg gat                                   33


<210>  54
<211>  34
<212>  DNA
<213>  artificial

<220>
<223>  artificial

<400>  54
ccggagctcc tattgccctt gttctggatg aggt                                  34


<210>  55
<211>  29
<212>  DNA
<213>  artificial

<220>
<223>  artificial

<400>  55
cgcggatcca tgggcgacca tgtggtggt                                        29


<210>  56
<211>  33
<212>  DNA
<213>  artificial

<220>
<223>  artificial

<400>  56
ccggagctcg acgctactga agtaactagc tat                                    33


<210>  57
<211>  1404
<212>  DNA
<213>  artificial

<220>
<223>  a homologous gene of AtTR1 from Zea mays L.(ZmTR1)


<220>
<221>  CDS
<222>  (158)..(1018)
<223>  the amino acid sequence encoded by ZmTR1

<400>  57
aaaaaaaaag ctcggaattt gtagtctcca agccgtgcga cgacccgacg ggaaggggaa      60

gagagggagg gccgccgctg aagtcgccgt cggtgaggag atcggttcct gcgtgcagag     120

gcggcaacaa gcgctgcaga gggaggaggg atttggg atg gct ggt gac gac cat      175
                                         Met Ala Gly Asp Asp His
                                         1               5

gtg gtg gtt gac gtg aac ggc tta gca aaa tcc aag gat gac ggg ggt      223
Val Val Val Asp Val Asn Gly Leu Ala Lys Ser Lys Asp Asp Gly Gly
            10                  15                  20

tcc aag aag ctg tct gag gat gca aat ggt tcg gct ccg gct gct gca      271
Ser Lys Lys Leu Ser Glu Asp Ala Asn Gly Ser Ala Pro Ala Ala Ala
        25                  30                  35

tct ttg tca gca gct gtt gac ttg gtc cag gag gag gag gag gag gag      319
Ser Leu Ser Ala Ala Val Asp Leu Val Gln Glu Glu Glu Glu Glu Glu
    40                  45                  50

ggt gat ggg gag gag cag ccg cta atc cag gcc gcg gag tgc cgc ata      367
Gly Asp Gly Glu Glu Gln Pro Leu Ile Gln Ala Ala Glu Cys Arg Ile
55                  60                  65                  70

tgc cag gag gaa gat agc gtg aag aac ctc gag aaa ccg tgt gct tgc      415
Cys Gln Glu Glu Asp Ser Val Lys Asn Leu Glu Lys Pro Cys Ala Cys
                75                  80                  85

agc ggc agt ctc aag tat gct cac aga gct tgc gtg caa cgc tgg tgc      463
Ser Gly Ser Leu Lys Tyr Ala His Arg Ala Cys Val Gln Arg Trp Cys
            90                  95                  100

aac gag aaa gga gac act act tgt gaa att tgt cat gag gaa tac aag      511
Asn Glu Lys Gly Asp Thr Thr Cys Glu Ile Cys His Glu Glu Tyr Lys
        105                 110                 115

cca ggt tac act gcg cca cct cgt gtt cag cca gat gag aca acc ata      559
Pro Gly Tyr Thr Ala Pro Pro Arg Val Gln Pro Asp Glu Thr Thr Ile
    120                 125                 130

gac att gat ggt gat ttg gtt atg gat ttg cga gac cca agg att ctt      607
Asp Ile Asp Gly Asp Leu Val Met Asp Leu Arg Asp Pro Arg Ile Leu
135                 140                 145                 150

gct gta gca gct gcc caa cat cgt ctt ctt gag gca gag tat gat ggt      655

Ala Val Ala Ala Ala Gln His Arg Leu Leu Glu Ala Glu Tyr Asp Gly
                155                 160                 165

tac gct agt aca gat gct agt ggt gct gca ttc tgc cgt tca gct gcg      703
Tyr Ala Ser Thr Asp Ala Ser Gly Ala Ala Phe Cys Arg Ser Ala Ala
            170                 175                 180

ctt att tta atg gca ctg ttg ctt cta agg cat gca ttg tct atg tca      751
Leu Ile Leu Met Ala Leu Leu Leu Leu Arg His Ala Leu Ser Met Ser
        185                 190                 195

gac aac gaa gga aac gat gat gac gct tct acc ata ttc tcg ctt ttt      799
Asp Asn Glu Gly Asn Asp Asp Asp Ala Ser Thr Ile Phe Ser Leu Phe
    200                 205                 210

ctg ctt cga gct gct ggg ttt ctg ctg ccg tgc tac atc atg gca tgg      847
Leu Leu Arg Ala Ala Gly Phe Leu Leu Pro Cys Tyr Ile Met Ala Trp
215                 220                 225                 230

ata ttc agt att ttg cat cgc cgt cga caa aga cag gaa gag gct gca      895
Ile Phe Ser Ile Leu His Arg Arg Arg Gln Arg Gln Glu Glu Ala Ala
                235                 240                 245

ctg gcg gct gca gag gtg gcc ttc atc cta cag tcg gcc agg ggc cgc      943
Leu Ala Ala Ala Glu Val Ala Phe Ile Leu Gln Ser Ala Arg Gly Arg
            250                 255                 260

gcc ctc cag ttt gcc atc gct cca gac tcc ccc gcc acc ccg cag cat      991
Ala Leu Gln Phe Ala Ile Ala Pro Asp Ser Pro Ala Thr Pro Gln His
        265                 270                 275

gag cca gtg tcg cag caa cag caa tag tggctgacaa ccacatcccc           1038
Glu Pro Val Ser Gln Gln Gln Gln
    280                 285

agtagttgct cacgactact gaccatacgt atctgtttcg ttttcttggg catcatactg   1098

agtcacaaag gttaagcctg ggcttctgcc tcccgtggcc agtttgggtg tcagtgtcac   1158

ccaaagttcg ttacacccga aaccttgtgc tttgtattaa accgagtgtg atccgagaat   1218

ataccaattt aatgtaaatt cattcttgtt cttactgcgg catcattatt aaccgttgta   1278

tgcactgtaa tgtacagtct ttcataatac tatttatttt tctatatggc atatttgata   1338

gatgtgaacg atgtggtgta tggaaattga aacctctatt gtttattgtg aaaaaaaaaa   1398

aaaaaa                                                              1404


<210>  58
<211>  286
<212>  PRT
<213>  artificial

<220>
<223>  Synthetic Construct

<400>  58

Met Ala Gly Asp Asp His Val Val Val Asp Val Asn Gly Leu Ala Lys
1               5                   10                  15


Ser Lys Asp Asp Gly Gly Ser Lys Lys Leu Ser Glu Asp Ala Asn Gly

            20                  25                  30

Ser Ala Pro Ala Ala Ala Ser Leu Ser Ala Ala Val Asp Leu Val Gln
        35                  40                  45

Glu Glu Glu Glu Glu Glu Gly Asp Gly Glu Glu Gln Pro Leu Ile Gln
    50                  55                  60

Ala Ala Glu Cys Arg Ile Cys Gln Glu Glu Asp Ser Val Lys Asn Leu
65                  70                  75                  80

Glu Lys Pro Cys Ala Cys Ser Gly Ser Leu Lys Tyr Ala His Arg Ala
                85                  90                  95

Cys Val Gln Arg Trp Cys Asn Glu Lys Gly Asp Thr Thr Cys Glu Ile
            100                 105                 110

Cys His Glu Glu Tyr Lys Pro Gly Tyr Thr Ala Pro Pro Arg Val Gln
        115                 120                 125

Pro Asp Glu Thr Thr Ile Asp Ile Asp Gly Asp Leu Val Met Asp Leu
    130                 135                 140

Arg Asp Pro Arg Ile Leu Ala Val Ala Ala Ala Gln His Arg Leu Leu
145                 150                 155                 160

Glu Ala Glu Tyr Asp Gly Tyr Ala Ser Thr Asp Ala Ser Gly Ala Ala
                165                 170                 175

Phe Cys Arg Ser Ala Ala Leu Ile Leu Met Ala Leu Leu Leu Leu Arg
            180                 185                 190

His Ala Leu Ser Met Ser Asp Asn Glu Gly Asn Asp Asp Asp Ala Ser
        195                 200                 205

Thr Ile Phe Ser Leu Phe Leu Leu Arg Ala Ala Gly Phe Leu Leu Pro
    210                 215                 220

Cys Tyr Ile Met Ala Trp Ile Phe Ser Ile Leu His Arg Arg Arg Gln
225                 230                 235                 240

Arg Gln Glu Glu Ala Ala Leu Ala Ala Ala Glu Val Ala Phe Ile Leu
                245                 250                 255

Gln Ser Ala Arg Gly Arg Ala Leu Gln Phe Ala Ile Ala Pro Asp Ser
            260                 265                 270

Pro Ala Thr Pro Gln His Glu Pro Val Ser Gln Gln Gln Gln
        275                 280                 285

<210>  59
<211>  18
<212>  DNA
<213>  artificial

<220>
<223>  artificial

<400>  59
ttgggatggc tggtgacg                                                    18


<210>  60
<211>  22
<212>  DNA
<213>  artificial

<220>
<223>  artificial

<400>  60
gtgagcaact actggggatg tg                                               22


<210>  61
<211>  25
<212>  DNA
<213>  artificial

<220>
<223>  artificial

<400>  61
ccggaattca tggctggtga cgacc                                            25


<210>  62
<211>  28
<212>  DNA
<213>  artificial

<220>
<223>  artificial

<400>  62
cccaagcttc tattgctgtt gctgcgac                                         28


<210>  63
<211>  52
<212>  PRT
<213>  artificial

<220>
<223>  AtTR1 critical peptides

<400>  63

Cys Arg Ile Cys Gln Glu Glu Asp Ser Thr Lys Asn Leu Glu Ala Pro
1               5                   10                  15


Cys Ala Cys Asn Gly Ser Leu Lys Tyr Ala His Arg Lys Cys Val Gln

20                          25                          30

Arg Trp Cys Asn Glu Lys Gly Asp Ile Thr Cys Glu Ile Cys His Gln
        35                          40                          45

Pro Tyr Gln His
    50

<210>  64
<211>  50
<212>  PRT
<213>  artificial

<220>
<223>  RcTR1 critical peptides

<400>  64

Cys Arg Ile Cys Gln Glu Glu Asp Ser Ile Asn Asn Leu Glu Ala Pro
1               5                   10                  15

Cys Ala Cys Ser Gly Ser Leu Lys Phe Ala His Arg Lys Cys Val Gln
                20                          25                          30

Arg Trp Cys Asn Glu Lys Gly Asp Ile Thr Cys Glu Ile Cys His Gln
        35                          40                          45

Pro Tyr
    50

<210>  65
<211>  51
<212>  PRT
<213>  artificial

<220>
<223>  ZmTR1 critical peptides

<400>  65

Cys Arg Ile Cys Gln Glu Glu Asp Ser Val Lys Asn Leu Glu Lys Pro
1               5                   10                  15

Cys Ala Cys Ser Gly Ser Leu Lys Tyr Ala His Arg Ala Cys Val Gln
                20                          25                          30

Arg Trp Cys Asn Glu Lys Gly Asp Thr Thr Cys Glu Ile Cys His Glu
        35                          40                          45

Glu Tyr Lys
    50

<210>  66
<211>  50

<212>  PRT
<213>  artificial

<220>
<223>  OsTR1-1 critical peptides

<400>  66

Cys Arg Ile Cys Gln Glu Glu Asp Leu Ala Lys Asn Leu Glu Ser Pro
1               5                   10                  15

Cys Ala Cys Ser Gly Ser Leu Lys Tyr Ala His Arg Glu Cys Val Gln
            20                  25                  30

Arg Trp Cys Asn Glu Lys Gly Asp Ile Ile Cys Glu Ile Cys His Val
        35                  40                  45

Ser Tyr
    50

<210>  67
<211>  48
<212>  PRT
<213>  artificial

<220>
<223>  OsTR1-2 critical peptides

<400>  67

Cys Arg Ile Cys Gln Glu Glu Asp Ser Ile Lys Asn Leu Glu Lys Pro
1               5                   10                  15

Cys Ala Cys Ser Gly Ser Leu Lys Tyr Ala His Arg Ala Cys Val Gln
            20                  25                  30

Arg Trp Cys Asn Glu Lys Gly Asp Ile Thr Cys Glu Ile Cys His Glu
        35                  40                  45

<210>  68
<211>  20
<212>  PRT
<213>  artificial

<220>
<223>  Conserved Domain of proteins that are able to enhance heat resistance
of plants or microorganisms

<220>
<221>  UNSURE
<222>  (7)..(7)
<223>  X represents 7-15 any amino acids

<220>
<221>  UNSURE
<222>  (12)..(12)
<223>  X represents 6 any amino acids

<220>
<221>  UNSURE
<222>  (16)..(16)
<223>  X represents 1 any amino acids

<220>
<221>  UNSURE
<222>  (20)..(20)
<223>  X represents 13-27 any amino acids

<400>  68

Cys Arg Ile Cys Gln Glu Xaa Pro Cys Ala Cys Xaa Ala His Arg Xaa
1               5                   10                  15

Cys Val Gln Xaa
            20

<210>  69
<211>  32
<212>  PRT
<213>  artificial

<220>
<223>  Conserved Domain of proteins that are able to enhance heat resistance
of plants or microorganisms

<220>
<221>  UNSURE
<222>  (9)..(9)
<223>  X represents 3-20 any amino acids

<220>
<221>  UNSURE
<222>  (12)..(12)
<223>  X represents 3-20 any amino acids

<220>
<221>  UNSURE
<222>  (17)..(17)
<223>  X represents 2 any amino acids

<220>
<221>  UNSURE
<222>  (21)..(21)
<223>  X represents 1 any amino acids

<220>
<221>  UNSURE
<222>  (25)..(25)
<223>  X represents 1 any amino acids

<220>
<221>  UNSURE
<222>  (32)..(32)
<223>  X represents 10-24 any amino acids

<400>  69

Cys Arg Ile Cys Gln Glu Glu Asp Xaa Asn Leu Xaa Pro Cys Ala Cys
1               5                   10                  15

Xaa Ser Leu Lys Xaa Ala His Arg Xaa Cys Val Gln Arg Trp Cys Xaa
            20                  25                  30

<210>  70
<211>  16
<212>  PRT
<213>  artificial

<220>
<223>  transmembrane domain

<220>
<221>  UNSURE
<222>  (2)..(2)
<223>  X represents 2-6 any amino acids

<220>
<221>  UNSURE
<222>  (6)..(6)
<223>  X represents 2-8 any amino acids

<220>
<221>  UNSURE
<222>  (10)..(10)
<223>  X represents 2-4 any amino acids

<220>
<221>  UNSURE
<222>  (13)..(13)
<223>  X represents 1-4 any amino acids
<220>
<221>  UNSURE
<222>  (16)..(16)
<223>  X represents 1-10 any amino acids

<400>  70

Ala Xaa Cys Arg Ser Xaa Leu Ile Leu Xaa Leu Leu Xaa Leu Arg Xaa
1               5                   10                  15

<210>  71
<211>  15
<212>  PRT
<213>  artificial

<220>
<223>  transmembrane domain

<220>
<221>  UNSURE
<222>  (2)..(2)
<223>  X represents 2-4 any amino acids

<220>
<221>  UNSURE
<222>  (4)..(4)
<223>  X represents 1-5 any amino acids

<220>

<221>  UNSURE
<222>  (9)..(9)
<223>  X represents 1-7 any amino acids

<220>
<221>  misc_feature
<222>  (15)..(15)
<223>  X represents 1-15 any amino acids

<400>  71

Leu Xaa Arg Xaa Gly Phe Leu Leu Xaa Tyr Ile Met Ala Trp Xaa
1               5                   10                  15